# EUROPEAN PATENT APPLICATION

(11) **EP 4 537 817 A1**
(43) Date of publication of application: **16.04.2025**
(21) Application number: 23819896.4
(22) Date of filing: 08.06.2023
(51) Int. Cl.: A61K 9/50, A61K 9/14, A61K 38/38, A61K 45/00, A61K 47/36, A61K 47/46, A61K 48/00, C08H 7/00, C08J 3/14

(54) **HOLLOW SPHERICAL PARTICLES**

(30) Priority: 08.06.2022 JP 2022093347
(71) Applicant: Kyoto University, Kyoto-shi, Kyoto 606-8501 (JP)
(72) Inventor: NISHIMURA, Hiroshi, Kyoto-shi, Kyoto 606-8501 (JP); MATOBA, Takahiro, Kyoto-shi, Kyoto 606-8501 (JP)
(74) Representative: Vossius & Partner Patentanwälte Rechtsanwälte mbB
(86) International application number: PCT/JP2023/021425
(87) International publication number: WO 2023/238916

(57) **Abstract**

An object is to provide a hollow spherical particle that has higher safety and can be prepared more easily. This object can be achieved by a hollow spherical particle that is a self-assembled body of a lignin-saccharide complex, the lignin-saccharide complex having at least one bond selected from the group consisting of an α-ether bond between lignin and polysaccharide, an α-ester bond between lignin and polysaccharide, and a γ-ester bond between lignin and polysaccharide.

## Description

### Technical Field

The present invention relates to a hollow spherical particle and the like.

### Background Art

Hollow spherical particles, such as microcapsules and nanocapsules, are used in a wide range of industries, including agricultural chemicals, detergents, fabric softeners, fragrances, cosmetics, pharmaceuticals, latent heat storage materials (PCM), textiles, paints, and adhesives. Encapsulating molecules, such as drugs, within particles can be expected to allow the drugs to act at the appropriate place and time, protect the drugs and disperse or accumulate them appropriately, and protect target objects and provide weather resistance by coating. Such capsules are widely used around the world; however, capsule materials derived from petroleum are difficult to recover after being released into the environment, and they have become a source of microplastics in soil, rivers, and oceans. Therefore, the development of naturally derived materials with a low environmental impact is an issue. Drug carriers (so-called drug delivery systems (DDS)) in the field of pharmaceuticals use liposomes, polymeric micelles, metal particles, etc. However, there are issues in biocompatibility, drug encapsulation, cell penetration and reachability, control of surface charge, sustained release properties, production costs, and structural stability. There is a need for high-performance, high-value-added drug carriers to solve these issues.

Hollow spherical particles composed of biologically derived organic polymers are good platforms for encapsulating or coating a variety of functional substances, such as lipids, polymers, proteins, metal nanoparticles, catalysts, magnetic materials, and carbon materials. They are expected to be applied in a wide range of fields, including environmental remediation by absorbing harmful substances, removal and detoxification of heavy metals, biosensors utilizing optical properties, catalytic functions, control of magnetic materials, energy storage, high-performance capacitors, protective coatings, and MRI and fluorescent imaging. In addition, by encapsulating capsules made of biologically derived biomass in matrixes such as resin, lighter weight and carbon neutralization are expected.

There have been reports of nanoparticles that use modified industrial lignin instead of petroleum-derived polymers (NPL 1); however, there are safety concerns because organic solvents that are harmful to living organisms are used in the production process thereof, and there are concerns about containing harmful aldehydes and sulfur atoms.

### Citation List

### Non-patent Literature

NPL 1: M. Lievonen et al. A simple process for lignin nanoparticle preparation, Green Chem. 2016, 18, 1416-1422 (https://pubs.rsc.org/en/content/articlelanding/2016/gc/c5gc01436 k)

### Summary of Invention

### Technical Problem

An object of the present invention is to provide a hollow spherical particle that has higher safety and can be prepared more easily. Another object of the present invention is to provide a hollow spherical particle that is smaller and more stable, and/or has the various effects described in the Background Art section, as well as high functionality and high added value as drug carriers, preferably in order to broaden the fields of application.

### Advantageous Effects of Invention

The present inventors conducted extensive research to achieve the above objects. As a result, the inventors found that a hollow spherical particle that is a self-assembled body of a lignin-saccharide complex, the lignin-saccharide complex having at least one bond selected from the group consisting of an α-ether bond between lignin and polysaccharide, an α-ester bond between lignin and polysaccharide, and a γ-ester bond between lignin and polysaccharide, could achieve the above objects. After further research based on this finding, the present invention has been accomplished. The present invention encompasses the following embodiments.

The present invention encompasses the following embodiments.

Item 1. A hollow spherical particle that is a self-assembled body of a lignin-saccharide complex, the lignin-saccharide complex having at least one bond selected from the group consisting of an α-ether bond, an α-ester bond, and a γ-ester bond between lignin and polysaccharide.

Item 2. The hollow spherical particle according to any one of the above Items, wherein the constituent ratio of polysaccharide to lignin in the lignin-saccharide complex, which is a ratio obtained by dividing the number of monomer units of polysaccharide by the number of monomer units of lignin, is 0.1 to 10.

Item 2A. An oil-in-water (O/W) emulsion, double emulsion, vesicle, micelle, capsule, lamellar structure, and single layer/multilayer structure, wherein the constituent ratio of saccharide to lignin in the lignin-saccharide complex is 1 to 100%, which is a percentage of the number of monomer units of saccharide to the number of monomer units of lignin.

Item 2B. A water-in-oil (W/O) emulsion, double emulsion, vesicle, reverse micelle, capsule, lamellar structure, and single layer/multilayer structure, wherein the constituent ratio of saccharide to lignin in the lignin-saccharide complex is 100% or more, which is a percentage of the number of monomer units of saccharide to the number of monomer units of lignin.

Item 2C. The hollow spherical particle according to any one of the above items, which is a population of hollow spherical particles composed of a lignin-saccharide complex and having an average particle size of 200 nm or more, preferably 250 nm or more, and more preferably 300 nm or more, and which has a positive average zeta potential at a pH of 7 and room temperature.

Item 2D. The hollow spherical particle according to any one of the above items, which is a population of hollow spherical particles composed of a lignin-saccharide complex and having an average particle size of less than 200 nm, and preferably 150 nm or less, and which has a negative average zeta potential at a pH of 7 and room temperature.

Item 3. The hollow spherical particle according to any one of the above Items, wherein the lignin-saccharide complex has a molecular weight (Mw) of 800 to 10,000 and a polydispersity (Mw/Mn) of 2.5 or less.

Item 4. The hollow spherical particle according to any one of the above Items, which has a number average particle size of 1 µm or less.

Item 5. The hollow spherical particle according to any one of the above Items, comprising an active ingredient.

Item 6. The hollow spherical particle according to Item 5, wherein the active ingredient is at least one member selected from the group consisting of dyes, agricultural chemicals, fertilizers, pharmaceuticals, cosmetic ingredients, fragrances, fluorescent agents, paints, proteins, antibodies, nucleic acids, vaccines, lipids, hormones, sugars, glycosides, surfactants, adhesives, vitamins, coenzymes, minerals, cells, organelles, low-molecular-weight compounds, high-molecular-weight compounds, metal ions, metal nanoparticles, metal complexes, magnetic materials, liposomes, and hydrogels.

Item 6A. A self-assembled body that has the ability to donate electrons to metal ions in an aqueous solution. A self-assembled body that can donate electrons equivalent to 1 µmol or more per mg of weight to gold(III) ions in an aqueous solution.

Item 7. The hollow spherical particle according to any one of the above Items, which exhibits long-wavelength fluorescence at 450 nm or more.

Item 8. A drug delivery or introducing agent comprising the hollow spherical particle according to any one of the above Items.

Item 9. The drug delivery or introducing agent according to Item 8, wherein the drug is nucleic acid.

Item 10. An ultraviolet absorber comprising the hollow spherical particle according to any one of the above Items.

Item 11. A light resistance imparting agent comprising the hollow spherical particle according to any one of the above Items.

Item 12. An anti-Stokes fluorescent agent comprising the hollow spherical particle according to any one of the above Items.

### Advantageous Effects of Invention

The present invention can provide a hollow spherical particle that is superior in biocompatibility, biodegradability, non-toxicity, and amphiphilicity, and that can be prepared more easily. The present invention can also provide sustained release properties by coating target objects or encapsulating and dispersing drugs, and allowing them to act at the appropriate place and time, positive and negative surface charges, cell penetration and reachability, stability in water and vacuum, light resistance, and selective identifiability by ultraviolet absorption and fluorescence absorption.

### Brief Description of Drawings

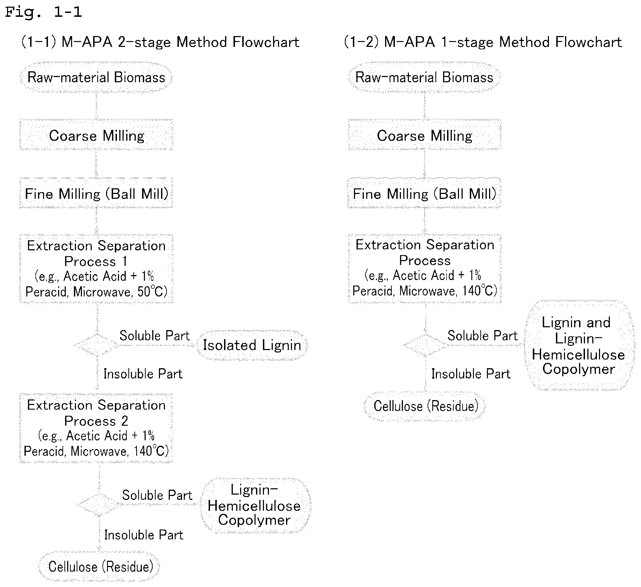

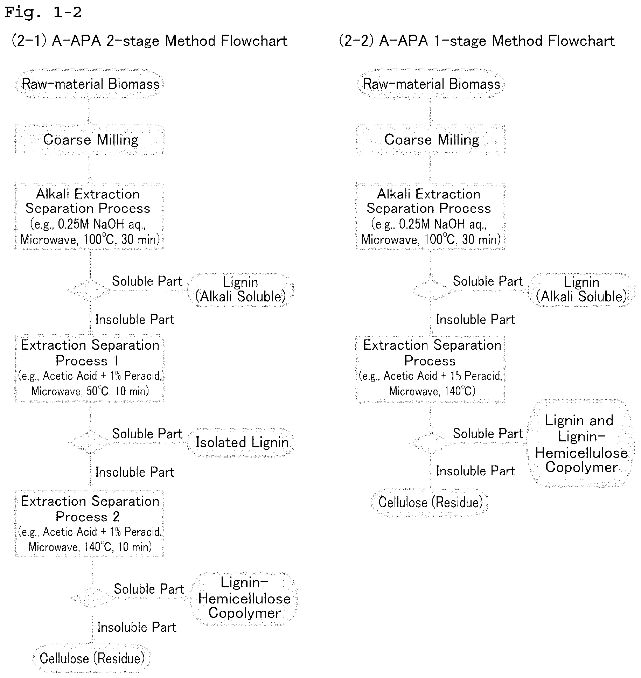

Fig. 1-1 shows flow charts of an M-APA method.
Fig. 1-2 shows flow charts of an A-APA method.
Fig. 2 shows microwave irradiation conditions for isolating lignocellulose components, power, temperature, and pressure profiles; and device specifications: Initiator+ 60 microwave synthesizer manufactured by Biotage, stirring speed: 600 rpm, Cooling: On, Absorption: High.
Fig. 3 is a two-dimensional NMR spectrum of a low-modified, high-purity lignin with a high ether-type content. This is a result of analysis of lignin isolated according to the M-APA method (microwave treatment at 50°C for 10 minutes with acetic acid-peracetic acid 1%) using *eucalyptus* as a starting material.
Fig. 4 shows the partial chemical structures of lignin interunit bonds and covalent bonds between lignin and hemicelluloses.
Fig. 5 shows two-dimensional NMR spectra a to d of lignin-hemicellulose complexes, and enlarged views a' to d' of interunit bonds and saccharide regions. Lignin-hemicellulose complexes obtained from hardwood (a, b) and softwood (c, d) according to the M-APA method were analyzed. The circles in the figure indicate LC bonds (ether type and ester type).
Fig. 6 is a synchro-fluorescence spectrum of APA lignin derived from bagasse (sugarcane) in the near-infrared wavelength region. This shows a result of synchro-scanning measurement for detecting a predetermined Stokes shift, that is, a result of the detection of fluorescence wavelength Em that is 80, 90, 150, 160, or 170 nm longer than excitation wavelength Ex (scanning of the excitation wavelength).
Fig. 7 shows a three-dimensional fluorescence spectrum of *Pinus densiflora* APA lignin in the near-infrared wavelength region. APA lignin obtained by treating wood flour of *Pinus densiflora* according to the M-APA method (microwaves at 50°C for 10 min with acetic acid + 0.2M hydrogen peroxide) was measured in an 80% acetonitrile solution (concentration: 0.1 wt%).
Fig. 8 shows UV-visible absorption spectra of commercial lignin (TCI alkaline lignin, Tokyo Chemical Industry Co., Ltd., blue line) and lignin (APA lignin, red line).

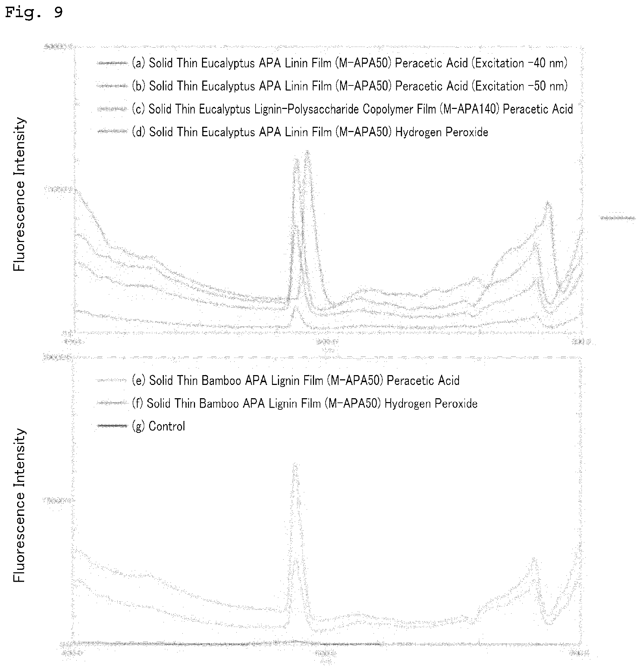

Fig. 9 shows synchronous anti-fluorescence spectra of solid thin lignin films and a solid thin lignin-saccharide complex film. Fig. 9 shows anti-fluorescence spectra (a to f) obtained by scanning an excitation wavelength and selectively detecting anti-Stokes-shifted fluorescence shorter than the excitation wavelength and control (g). The anti-Stokes shift was an excitation wavelength minus 50 nm; minus 40 nm only in spectrum (b). From the comparison between spectra (b) and (d) and the comparison between spectra (e) and (f), the anti-fluorescence intensity was found to be higher when peracetic acid, rather than hydrogen peroxide, was used as an oxidizing agent. The comparison of spectra (b) and (c) indicates that the APA lignin exhibits a higher anti-fluorescence intensity than the lignin-saccharide complex.
Fig. 10 shows UV absorption spectra of lignin and a lignin-hemicellulose complex. The vertical axis indicates absorbance, and the horizontal axis indicates wavelength (nm). For an explanation of the legend, see Table 7.
Fig. 11-1 shows a bright-field image of the hollow spherical particles obtained in Reference Test Example 5.
Fig. 11-2 shows scanning electron microscopy (SEM) photographs of the lignin capsules (a and b) and lignin-saccharide complex capsules (c) obtained in Example 1.
Fig. 12-1 shows a fluorescence image of the hollow spherical particles obtained in Reference Test Example 6.
Fig. 12-2 shows micrographs of the lignin-saccharide complex capsules encapsulating fluorescent dye DAPI obtained in Example 1. These are fluorescence micrographs (A1 and A2), bright-field micrographs (B1 and B2), and SEM photographs (C1, C2, C3, and C4). The fluorescent micrographs show drug encapsulation. The SEM photographs show that the hollow spherical particles do not break and can maintain their shape even in a vacuum, indicating the stability of the particle structure.
Fig. 13 shows the relative average particle size of the hollow spherical particles obtained in Example 2. The horizontal axis indicates the time elapsed from immediately after the preparation of the hollow spherical particles.
Fig. 14 shows the relative average particle size of the hollow spherical particles obtained in Example 2. The horizontal axis indicates the temperature during heating and stirring.
Fig. 15 shows the particle size and zeta potential distribution of eucalyptus lignin capsules (LP, top) and eucalyptus lignin-saccharide complex capsules (LHP, bottom) (Example 1).
Fig. 16 shows the 3D fluorescence spectra of lignin-saccharide complex capsules. The vertical axis indicates the excitation wavelength (nm), and the horizontal axis indicates the fluorescence wavelength (nm).
Fig. 17 shows the UV-visible absorption spectrum of the reaction solution (Reference Test Example 3).
Fig. 18 shows the pore passing time and size plot of sub-microparticles measured by the electrical resistance nanopulse method (qNANO) (Example 5-2-1).
Fig. 19 shows SPM image results (left: 10.00 µm x 10.00 µm analysis, right: 2.50 µm x 2.50 µm analysis results) (Example 5-2-2).
Fig. 20 shows the results of SPM 3D shape analysis (left: image analysis, right: 3D shape analysis) (Example 5-2-2).
Fig. 21 shows the results of SPM physical property analysis (left: shape analysis, center: adhesion force analysis, right: hardness analysis) (Example 5-2-2).
Fig. 22 shows the results of 3D real surface view microscope observation of lignin-saccharide complex fine particles (LCC lignin fine particles) (Example 5-2-2).
Fig. 23 shows image data of LCC nanoparticles taken with an interference microscope (Example 5-2-2).

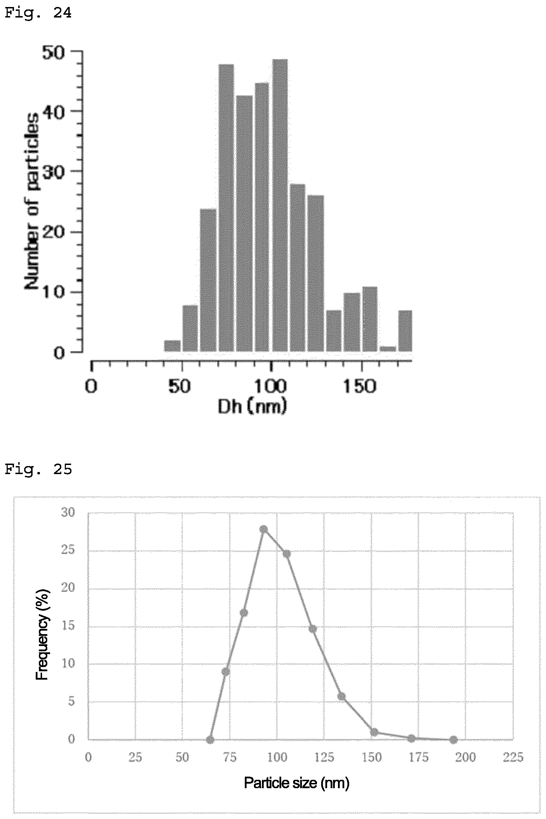

Fig. 24 shows the particle size histogram of LCC nanoparticles taken with an interference microscope (Example 5-2-2).
Fig. 25 shows the results of evaluating the particle size of LCC nanoparticles by DLS (Example 5-2-2).

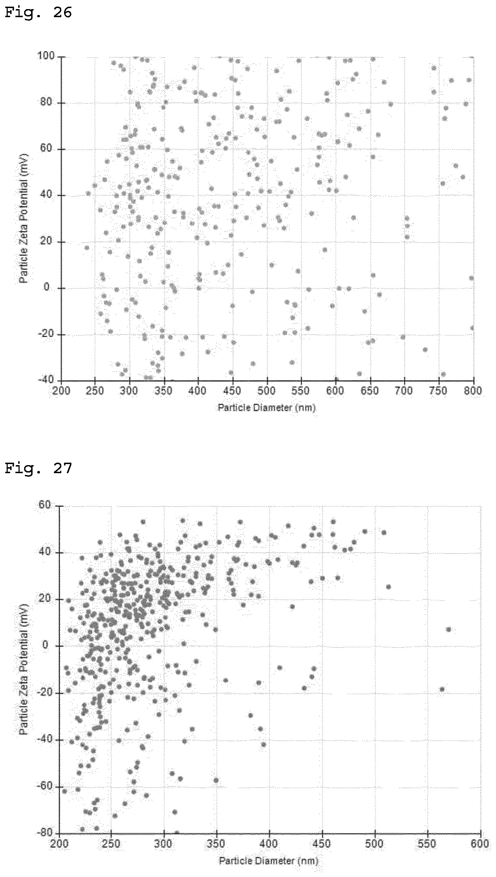

Fig. 26 shows the results of measuring the zeta potential of pH3 lignin fine particles by the electrical resistance nanopulse method (qNANO) (Example 5-2-3-1).
Fig. 27 shows the results of measuring the zeta potential of pH5 lignin fine particles by the electrical resistance nanopulse method (qNANO) (Example 5-2-3-1).
Fig. 28 shows the results of measuring the zeta potential of pH7 lignin fine particles by the electrical resistance nanopulse method (qNANO) (Example 5-2-3-1).
Fig. 29 shows the results of measuring the zeta potential of pH9 lignin fine particles by the electrical resistance nanopulse method (qNANO) (Example 5-2-3-1).
Fig. 30 shows the results of measuring the zeta potential of p11 lignin fine particles by the electrical resistance nanopulse method (qNANO) (Example 5-2-3-1).
Fig. 31 shows the results of measuring the zeta potential of pH3 LCC lignin fine particles by the electrical resistance nanopulse method (qNANO) (Example 5-2-3-1).
Fig. 32 shows the results of measuring the zeta potential of pH5 LCC lignin fine particles by the electrical resistance nanopulse method (qNANO) (Example 5-2-3-1).
Fig. 33 shows the results of measuring the zeta potential of pH7 LCC lignin fine particles by the electrical resistance nanopulse method (qNANO) (Example 5-2-3-1).
Fig. 34 shows the results of measuring the zeta potential of pH9 LCC lignin fine particles by the electrical resistance nanopulse method (qNANO) (Example 5-2-3-1).
Fig. 35 shows the results of measuring the zeta potential of pH11 LCC lignin fine particles by the electrical resistance nanopulse method (qNANO) (Example 5-2-3-1).
Fig. 36 shows the results of measuring the 3D fluorescence excitation spectrum of LCC lignin fine particles (Example 5-2-4).
Fig. 37 shows the results of measuring the 3D fluorescence excitation spectrum of eosin complex LCC lignin fine particles (Example 5-2-4).
Fig. 38 shows the results of evaluating the particle size of diethyl sebacate complex fine particles using DLS (Example 5-2-5).
Fig. 39 shows the results of measuring the fluorescence excitation spectrum of diethyl sebacate complex fine particles (Example 5-2-5).
Fig. 40 shows a photograph of water-in-1-butanol (W/O) lignin-saccharide complex hollow spherical particles (Test Example X).

### Description of Embodiments

In the present specification, the terms "comprise," "contain," and "include" include the concepts of comprising, containing, including, consisting essentially of, and consisting of.

In one embodiment, the present invention relates to a hollow spherical particle that is a self-assembled body of a lignin-saccharide complex (which may be referred to as "the lignin-saccharide complex of the present invention" in the present specification), the lignin-saccharide complex having at least one bond selected from the group consisting of an α-ether bond between lignin and polysaccharide, an α-ester bond between lignin and polysaccharide, and a γ-ester bond between lignin and polysaccharide (which may be referred to as "the hollow spherical particle of the present invention" or "self-assembled body" in the present specification). The saccharide in the lignin-saccharide complex of the present specification is derived from polysaccharide and contains at least one monomer unit as the saccharide. The details are described below.

### 1. Lignin-Saccharide Complex Isolation Method

The lignin-saccharide complex of the present invention can be obtained by a method for isolating lignin from plant biomass, the method comprising step (A) of bringing a solution containing an organic acid and a peracid into contact with plant biomass (which may be referred to as "the isolation method of the present invention" in the present specification). The details are described below.

The isolation method of the present invention comprises step (A), whereby a high-quality (low-condensation) complex of lignin and polysaccharide with a high content of the β-O-4 ether type structure among all of the binding modes between the monolignols can be obtained.

The plant biomass may be any plant biomass that contains a lignin-saccharide complex. Examples of the plant biomass include a plant body itself and a mechanically processed product of a plant body.

Examples of the plant body include softwood materials, hardwood materials, and non-wood materials. Specific examples include softwood materials of, for example, *Cryptomeria japonica,* pine, *Picea jezoensis, Larix kaempferi, Pinus thunbergii, Abies sachalinensis, Pinus parviflora, Taxus cuspidata, Thuja standishii, Picea polita, Picea alcokiana, Podocarpus macrophyllus, Abies firma, Chamaecyparis pisifera, Pseudotsuga japonica, Thujopsis dolabrata* var. *dolabrata, Thujopsis dolabrata* var. *hondae, Tsuga sieboldii, Tsuga diversifolia, Chamaecyparis obtusa, Taxus cuspidata, Cephalotaxus harringtonia, Picea jezoensis* var. *hondoensis,* yellow cedar (*Callitropsis nootkatensis*), Lawson cypress (*Chamaecyparis lawsoniana*), Douglas fir (*Pseudotsuga menziesii*), Sitka spruce (*Picea sitchensis*), *Pinus radiata,* eastern spruce, eastern white pine, western larch, western fir, western hemlock, and tamarack; hardwood materials of, for example, *Populus Tremuloides,* American black cherry, *Liriodendron tulipifera,* walnut, kaba-zakura, *Zelkova serrata,* sycamore, silver cherry, *Fraxinus mandshurica, Tectona grandis,* Chinese elm, Chinese maple, *Quercus*, *Fagus crenata*, hard maple, hickory, *Carya illinoinensis*, *Fraxinus americana*, white oak, white birch, red oak, acacia, and eucalyptus; and non-wood materials of, for example, *Oryza sativa*, *Saccharum officinarum*, barley, wheat, maize, pineapple, oil palm, *Hibiscus cannabinus*, cotton, alfalfa, *Phleum pratense,* bamboo, *Sasa,* bamboo, and sugar beet.

Examples of the mechanically processed product of a plant body include logs, rectangular lumbers, boards, solid wood, wood materials, engineered wood, laminated veneer lumbers, plywood, wooden board, particle board, fiber board, wood chips, particulate wood materials (e.g., chips, particles, and wood flour), fibrous wood materials, compressed materials, and crushed materials.

The plant biomass is preferably wood flour from the viewpoint of being able to improve the yield. The volume average particle size of wood flour is, for example, 500 µm or less, preferably 200 µm or less, more preferably 100 µm or less, even more preferably 50 µm or less, and still more preferably 20 µm or less. The lower limit of the average particle size may be any value and can be, for example, 1 µm, 2 µm, or 5 µm. By using wood flour with a relatively small particle size, a high yield can be achieved without the alkali treatment described below.

Wood flour can be obtained by pulverizing plant biomass. Pulverization can be performed following or in accordance with known methods, for example, by using various types of mills (e.g., ball mills and mixer mills).

The plant biomass is preferably an alkali-treated material of a plant body or an alkali-treated material of a mechanically processed product of a plant body from the viewpoint of being able to improve the yield. According to the present invention, the use of the alkali-treated material makes it possible to efficiently obtain the target product without performing pulverization treatment (even when the specific surface area is relatively large, as is the case with wood chips). The alkali-treated material can be obtained by subjecting a plant body or a mechanically processed product thereof to alkali treatment. Alkali treatment can improve the yield by causing loosening of the plant cell wall structure (reducing intermolecular interactions and cleaving hydrogen bonds) and by cleaving intramolecular ester bonds. This allows a high yield to be achieved without physical pulverization of plant biomass or with a low degree of physical pulverization of plant biomass, thereby reducing process costs. Furthermore, the yield can be improved by 1.2 to 2 times. Moreover, the cellulose fibers obtained through the alkali treatment have no significant decrease in crystallinity due to fine grinding, and retain a clear cellulose Iβ structure.

Specifically, the alkali treatment is preferably, for example, step (X) of bringing the plant body or the mechanically processed product thereof into contact with an alkaline solution.

The alkaline solution may be any alkaline solution as long as the above object can be achieved. For example, the alkaline solution can be a solution with a pH of 12 to 15. The pH of the solution is preferably 13 to 14. The alkaline solution can also be, for example, a solution containing 0.3 to 2 mass% (preferably 0.7 to 1.5 mass%) of an alkali metal hydroxide (sodium hydroxide, potassium hydroxide, etc.).

The amount of the alkaline solution for use may be any amount as long as the above object can be achieved. The alkaline solution can be used in an amount of, for example, 3 to 20 mL, and preferably 5 to 12 mL, per 1 g of the plant body or the mechanically processed product thereof.

The contact mode between the plant body or the mechanically processed product thereof and the alkaline solution may be any mode. From the viewpoint of, for example, treatment efficiency, it is preferable to immerse the plant body or the mechanically processed product thereof in the alkaline solution.

The temperature of the alkaline solution may be any temperature as long as the above object can be achieved. The temperature of the alkaline solution can be, for example, 20 to 150°C, preferably 50 to 120°C, and more preferably 80 to 120°C. When heating is performed, examples of the heating method include an internal heating method using microwave heating, and an external heating method using an oil bath etc. Preferred is an internal heating method using microwave heating.

The treatment time in step (X) is not limited as long as the above object can be achieved. The treatment time is, for example, 5 to 120 minutes, and preferably 15 to 60 minutes.

After step (X), the soluble part and the insoluble part are separated, and the insoluble part can be subjected to step (A) as an alkali-treated material of a plant body or an alkali-treated material of a mechanically processed product of a plant body.

Plant biomasses can be used singly or in a combination of two or more.

The solution containing an organic acid and a peracid (an organic acid-peracid solution) may be any solution that contains an organic acid and a peracid, and that is capable of extracting and solubilizing at least one member selected from the group consisting of lignin, hemicelluloses, and lignin-saccharide complexes.

Examples of the organic acid include, but are not limited to, acetic acid, formic acid, glyoxylic acid, maleic acid, propionic acid, folic acid, isobutyric acid, valeric acid, isovaleric acid, pyruvic acid, oxalic acid, malonic acid, succinic acid, glutaric acid, ketoglutaric acid, adipic acid, lactic acid, tartaric acid, fumaric acid, oxaloacetic acid, malic acid, isocitric acid, citric acid, benzoic acid, phthalic acid, isophthalic acid, terephthalic acid, hemimellitic acid, trimellitic acid, trimesic acid, mellophanic acid, prehnitic acid, pyromellitic acid, mellitic acid, methanesulfonic acid, benzenesulfonic acid, p-toluenesulfonic acid, camphorsulfonic acid, p-toluenesulfinic acid, and benzenesulfinic acid. Among these, preferred are acetic acid, formic acid, glyoxylic acid, 3-oxopropanoic acid, 2-methyl-3-oxopropanoic acid, and the like, more preferred are acetic acid, formic acid, glyoxylic acid, and the like, and particularly preferred is acetic acid.

The organic acids can be used singly or in a combination of two or more.

The peracid may be any acid that contains a hydroperoxide group (-O-OH). Examples of peracids include organic peroxo acids such as percarboxylic acid, and persulfuric acid, percarbonic acid, perphosphoric acid, and peroxo-perhalogenic acid. Examples of percarboxylic acids include peracetic acid, performic acid, perbenzoic acid, and metachloroperbenzoic acid. Examples of peroxo-perhalogenic acid include peroxo-perchloric acid, peroxo-perbromic acid, and peroxo-periodic acid. In addition to the above, examples of peracids include hydrogen peroxide, lithium peroxide, sodium peroxide, potassium peroxide, sodium percarbonate, urea peroxide, sodium perborate, tert-butyl hydroperoxide, cumene hydroperoxide, di-tert-butyl peroxide, dimethyldioxirane, acetone peroxide, methyl ethyl ketone peroxide, and hexamethylene triperoxide diamine. Among these, preferred are organic peroxo acids, hydrogen peroxide, sodium percarbonate, urea peroxide, sodium perborate, and the like, more preferred are percarboxylic acids (in particular, peracetic acid), hydrogen peroxide, and the like, and particularly preferred is hydrogen peroxide.

The peracids can be used singly or in a combination of two or more.

The organic acid content in the organic acid-peracid solution is, for example, 5 to 30 mol/L, preferably 8 to 25 mol/L, and more preferably 10 to 20 mol/L. The organic acid content in the organic acid-peracid solution is preferably 3 to 10 g, more preferably 4 to 8 g, and even more preferably 5 to 7 g, per 1 g of the plant biomass to be brought into contact with the organic acid-peracid solution.

The peracid content in the organic acid-peracid solution is, for example, 0.05 to 3 mol/L, preferably 0.08 to 2.5 mol/L, and more preferably 0.1 to 2 mol/L. The peracid content in the organic acid-peracid solution is preferably 0.03 to 0.1 g, more preferably 0.04 to 0.08 g, and even more preferably 0.05 to 0.07 g, per 1 g of the plant biomass to be brought into contact with the organic acid-peracid solution.

The contact mode between the plant biomass and the organic acid-peracid solution may be any mode. From the viewpoint of, for example, treatment efficiency, it is preferable to immerse the plant biomass in the organic acid-peracid solution.

The temperature of the organic acid-peracid solution may be any temperature as long as the lignin-saccharide complex can be extracted and solubilized. The temperature of the organic acid-peracid solution is, for example, 60 to 160°C. By setting the temperature to be approximately within the above range, a high-quality (low-condensation) complex of lignin and polysaccharide with a high content of the β-O-4 ether type structure among all of the binding modes between the monolignols can be obtained. In step (A), a complex of lignin and polysaccharide tends to be obtained when treated at a high temperature, while lignin alone tends to be obtained when treated at a low temperature. For example, when the starting material is wood-based biomass, lignin alone is more likely to be selectively obtained by performing step (A) using the organic acid-peracid solution at a temperature of, for example, 90°C or lower (preferably 40 to 90°C, more preferably 40 to 70°C, and even more preferably 40 to 60°C). When step (A) is performed in an organic acid-peracid solution, for example, at more than 90°C and 160°C or less (preferably 100 to 160°C, and more preferably 120 to 160°C), a complex of lignin and polysaccharide is likely to be obtained. For example, when the starting material is herbaceous biomass, lignin alone is more likely to be selectively obtained by performing step (A) using the organic acid-peracid solution at a temperature of, for example, 60°C or lower (preferably 15 to 60°C). When step (A) is performed in an organic acid-peracid solution, for example, at more than 60°C and 150°C or less (preferably 70 to 150°C), a complex of lignin and polysaccharide is likely to be obtained. When heating is performed, examples of the heating method include an internal heating method using microwave heating, and an external heating method using an oil bath etc. Preferred is an internal heating method using microwave heating. Rapid internal heating with microwaves enables quick and efficient pretreatment.

The treatment time in step (A) is not limited as long as the above object can be achieved. The treatment time is, for example, 1 to 60 minutes, preferably 1 to 30 minutes, and more preferably 5 to 20 minutes.

In step (A), microwave treatment is preferably performed while the biomass is immersed in the solution. This improves the lignin yield. The conditions for microwave treatment can be set as appropriate. For example, a commercially available microwave synthesizer can be used while stirring the solution. The microwave treatment time can be, for example, 1 to 30 minutes, and preferably about 10 minutes. The isolation method of the present invention can also easily be constructed as a continuous system as well as a batch system, since the microwave treatment, when performed, requires only a relatively short irradiation time.

In step (A), it is preferable to irradiate the plant biomass with microwaves or ultrasonic waves from the viewpoint of being able to promote penetration of the solution into the internal pits inside the plant cell wall and being able to attempt to improve the yield. The irradiation time is the same as described above.

Step (A) may be performed in multiple stages while changing the conditions as necessary. For example, step (A) can be performed while changing the temperature of the solution, in which case it is preferable that the temperature of the solution is higher in the next step when comparing a particular step with the next step. For example, step (A) may include step (A1) of bringing a solution containing organic acid and peracid at 90°C or less into contact with plant biomass, and/or step (A2) of bringing a solution containing organic acid and peracid at more than 90°C and 160°C or less into contact with plant biomass. The "plant biomass" in step (A2) contains the insoluble part obtained in step (A1).

Step (A1) enables selective extraction and solubilization of lignin alone, and step (A2) enables extraction and solubilization of the lignin-saccharide complex. Therefore, lignin alone and the lignin-saccharide complex can be efficiently obtained by performing step (A1) and step (A2) in this order.

After step (A), a separating solvent is added, if necessary, and then the soluble part and insoluble part are separated. The method of separating the soluble part from the insoluble part may be any method. Examples of usable methods include decantation, spin-down, and filtration. The separating solvent may be dioxane, ethanol, acetone, acetonitrile, DMSO, DMF, NMI, ethyl acetate, methanol, butanol, various other alcohols, acetic acid, water, and mixed solvents thereof. By using these, lignin and a lignin-hemicellulose complex are separated. Preferred are organic solvents of acetone, ethanol, and acetonitrile, and mixtures of 10 to 20 v/v% organic solvents in water, from the viewpoint of extraction efficiency, selectivity, and environmental burden.

A lignin-hemicellulose complex with high purity can be obtained by using a water-free extraction solvent (acetic acid-peracetic acid) and a separating solvent. When a water-containing solvent is used, a part of the hemicellulose component, which is polysaccharide, is also obtained at the same time. The hydrolysis reaction can be carried out in the presence of an acid or an alkali using a catalyst that accelerates the reaction.

The soluble part obtained in step (A) contains a lignin-saccharide complex. Thus, the soluble part can be obtained as the lignin-saccharide complex, either as is or through other treatments, if necessary.

Examples of the other treatments include the following. After step (A), water can be removed by evaporation and/or lyophilization, if necessary, to obtain the target product as a dry powder. If desalting is necessary, the concentrated solution may be extracted, for example, with alcohol or acetone, and the solvent may be distilled off. This allows the target product to be obtained as a dry powder.

In the case of selectively obtaining a lignin-saccharide complex, for example, the lignin-saccharide complex can be obtained from the soluble part obtained in step (A2), which uses the insoluble part obtained in step (A1) as plant biomass.

As a novel process for producing paper pulp with low energy and no harmful effluents, the present invention can provide a novel method for pretreating plant biomass, the method being a low-temperature, mild process with sufficiently delignified polysaccharides, so that the recovered product contains a further reduced amount of inhibitory substances that cause problems in ethanol fermentation.

### 2. Lignin-Saccharide Complex

The isolation method of the present invention makes it possible to obtain a lignin-saccharide complex characterized by at least having at least one bond selected from the group consisting of an α-ether bond between lignin and polysaccharide, an α-ester bond between lignin and polysaccharide, and a γ-ester bond between lignin and polysaccharide.

### 2-1. Lignin

The lignin of the present invention refers to lignin in a state isolated from biomass (including natural lignin). The binding modes in natural lignin are diverse. Representative examples of the binding modes in natural lignin include the following.

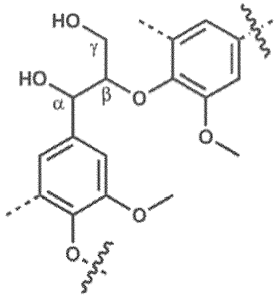

The above is called a β-O-4 bond.

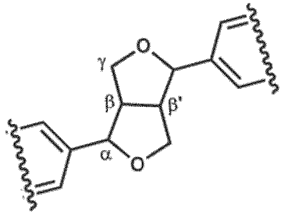

The above is called a resinol (β-β) bond.

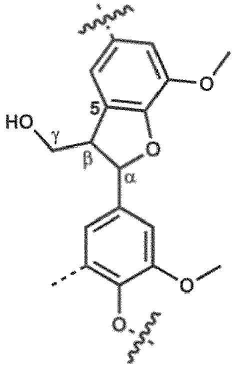

The above is called a phenylcoumaran (β-5) bond.

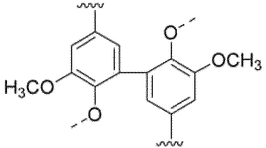

The above is called a 5-5 bond. 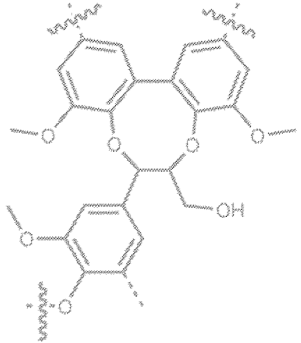 The above is called a dibenzodioxin (DBDO 5-5/4-O-β) bond.

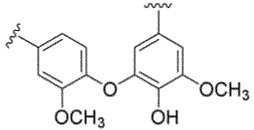

The above is called a 4-O-5 bond.

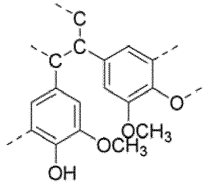

The above is called a β-1 bond.

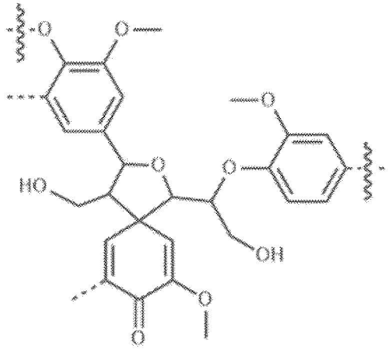

The above is called a spirodienone bond.

In natural lignin, the content of the β-O-4 ether type structure is highest among all of the binding modes between the monolignols, although it also varies depending on plant species. For example, in softwood, the content of the same is considered to be 40 to 60%. Further, in hardwood, for example, the content of the same in eucalyptus is considered to be about 45%.

In the lignin of the present invention, the content of the β-O-4 ether type structure is 50% or more, preferably 60% or more, more preferably 65 to 85%, and even more preferably 70 to 80%, among all of the binding modes between the monolignols.

In the lignin of the present invention, the content (%) of the β-O-4 ether type structure among all of the binding modes between the monolignols is preferably 1.2x% or more, more preferably 1.4x% or more, and even more preferably 1.6x% or more, with the content of the same in the lignin in the biomass used as a starting material being defined as x%. In the above, the upper limit is preferably 2x%.

The content of the β-O-4 ether type structure among all of the binding modes between the monolignols in lignin is specifically measured as follows. A sample is dissolved in deuterated dimethyl sulfoxide (DMSO-d₆) to 1 to 10 wt%, and the NMR spectrum is obtained using a Bruker Avance III 600 MHz NMR Spectrometer equipped with a low-temperature probe. The content of the β-O-4 ether type structure can be estimated from the signal volume in the HSQC (Heteronuclear single-quantum correlation spectroscopy) spectrum. The quantitative value in the two-dimensional NMR method is corrected according to a document (H. Okamura, H. Nishimura, T. Nagata, T. Kigawa, T. Watanabe, and M. Katahira, Accurate and molecular-size-tolerant NMR quantitation of diverse components in solution, Scientific Reports, 6, 21742, 2016) to calculate the accurate content.

In the lignin of the present invention, the constituent ratio of biphenyl-type lignin with a condensed structure is preferably 5% or less.

The lignin of the present invention has a number average molecular weight (Mn) of preferably 0.7 to 9 kDa, more preferably 1 to 5 kDa, and even more preferably 1.5 to 4 kDa.

The lignin of the present invention has a weight average molecular weight (Mw) of preferably 0.8 to 10 kDa, more preferably 1.5 to 8 kDa, and even more preferably 2 to 6 kDa.

In the present invention, the number average molecular weight (Mn) and weight average molecular weight (Mw) of lignin are specifically measured as follows. The molecular weight of lignin can be determined by size exclusion chromatography (SEC), i.e., gel permeation chromatography (GPC). A sample is dissolved in a solution of acetic anhydride and a base (e.g., pyridine) and reacted for 3 to 6 hours to obtain an acetylated product. The acetylated product is dissolved in tetrahydrofuran (THF) to a concentration of 2.0 mg/mL, and separation is performed by high-performance liquid chromatography. The columns for use are HZ-M columns (Tosoh Corporation, 150 mm × 4.6 mm id, 4 µm, three columns connected in series). The analysis is performed by injecting 10 µL of the sample at a column temperature of 40°C using tetrahydrofuran (THF) as the mobile phase at a flow rate of 0.35 mL/min. A mass calibration curve is prepared using standard polystyrene, Tosoh PStQuick C (molecular weights (MW): 2,110,000, 427,000, 37,900, and 5970), pinoresinol (MW: 352), and vanillin (MW: 152) to determine the Mw and Mn of the sample.

The lignin of the present invention has a polydispersity (Mw/Mn) of preferably 2.5 or less, and more preferably 2 or less.

### 2-2. Lignin-Saccharide Complex

The lignin-saccharide complex of the present invention refers to a lignin-saccharide complex isolated from biomass.

Examples of polysaccharides include xylan, mannan, glucomannan, galactoglucomannan, glucuronoxylan, arabinoglucuronoxylan, and the like. The main component of hemicellulose is glucomannan in softwood, glucuronoxylan in hardwood, and arabinoxylan in herbaceous plants. In the above, xylose, arabinose, glucose, mannose, galactose, and uronic acid are contained as monomers. In addition, acetyl and methoxy groups are contained in the side chain. In herbaceous plants, hemicellulose contains ferulic acid and diferulic acid bound to the arabinose side chains of arabinoxylan.

The lignin-saccharide complex of the present invention is a lignin-saccharide complex that can be uniformly dispersed in a separating solvent, and is characterized by a polymer structure in which lignin and polysaccharide are copolymerized by covalent bonds. The lignin-saccharide complex of the present invention is isolated by cutting, by hydrolysis, a small portion of the bonds (10% or less of the bonds) in the hemicellulose main chain of the lignin-saccharide complex structure in plant cell walls. It is possible to suppress over-degradation, side reactions, and degradation into smaller molecules by performing hydrolysis under mild conditions.

The lignin-saccharide complex of the present invention is characterized by having at least one bond selected from the group consisting of an α-ether bond (LC α-ether bond) between lignin and polysaccharide, an α-ester bond (LC α-ester bond) between lignin and polysaccharide, and a γ-ester bond (LC γ-ester bond) between lignin and polysaccharide.

In the lignin-saccharide complex of the present invention, the α-ether bond (LC α-ether) between lignin and polysaccharide, which is the copolymerization site, is preferably concentrated 1.2 times or more compared to the original binding unit constituent ratio of the raw material biomass.

In the biomass, lignin and polysaccharide are also bound by non-covalent bonds. Non-covalent bonds are due to hydrophobic interactions, hydrogen bonds, electrostatic interactions, and entanglement of polymer chains. Accordingly, previous studies have not provided clear evidence that lignin and polysaccharide, which are polymers with different properties, form copolymer complexes through covalent bonds in lignin-saccharide complexes. In addition, conventional methods for preparing lignin-saccharide complexes involve multiple steps, resulting in low yield (less than 5%) and low purity.

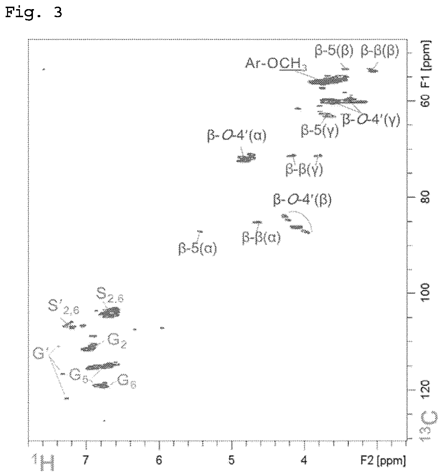

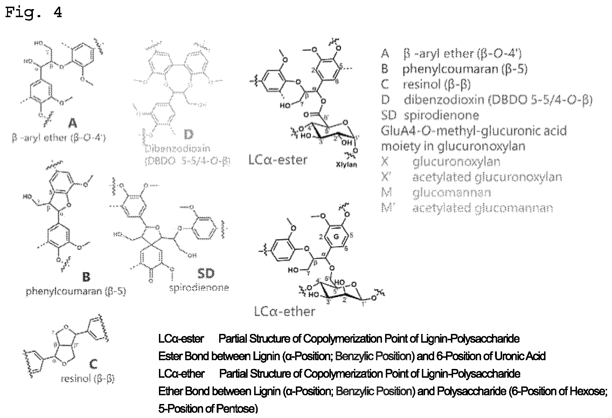

Clear evidence of copolymerization sites (covalent bonds) in the lignin-hemicellulose copolymer can be confirmed by signal assignment using the HSQC method, which is two-dimensional NMR (Figs. 3 and 4). Further clear evidence can be confirmed by continuous correlation analysis using HMBC and TOCSY-HSQC methods. For more detail, reference can be made to the following documents: Nishimura et al., Direct evidence for α ether linkage between lignin and carbohydrates in wood cell walls, Sci Rep 2018; and Nishimura et al., Direct evidence of α ester linkages between lignin and glucuronoxylan that reveal the robust heteropolymeric complex in plant cell walls, Research Square https://doi.org/10.21203/rs.3.rs-1327348/v1.

### LC α-ether

C-H correlation signal at the α-position of lignin (LC ether, δH/δC = 4.50 ±0.15 ppm / 80.1 ±1 ppm region) derived from the ether bond between the hydroxyl group at the 6-position of a hexose or the 5-position of a pentose (e.g., 6-position of mannose, glucose, or galactose, and 5-position of arabinose) and the α-position (benzyl position) of lignin, and the hydroxyl group at the 2- or 3-position of a hexose or pentose (e.g., 2-and 3-positions of xylose, mannose, glucose, galactose, or arabinose) (LC ether, δH/δC = 4.95 ±0.2 ppm / 81.5 ±1.5 ppm region).

### LC α-ester

C-H correlation signal at the α-position of lignin (LC α-ester, δH/δC = 5.88 ±0.15 ppm / 74.0 ±1.5 ppm region) derived from the ester bond between the carboxy group at the 6-position of glucuronic acid (uronic acid) and the α-position (benzyl position) of lignin.

### LC γ-ester

C-H correlation signal at the γ-position of lignin (LC γ-ester, δH/δC = 4.0 ±0.15 ppm, 4.4 ±0.15 ppm, / 64.8 ±1.5 ppm region) derived from the ester bond between the carboxy group at the 6-position of glucuronic acid (uronic acid) and the γ-position of lignin.

The lignin-saccharide complex of the present invention is characterized by having a solubility in 60 to 80% (e.g., 60% or 80%) ethanol at 25°C of 2 w/v% or more. For this reason, by using the lignin-saccharide complex of the present invention, a hollow spherical particle described below can be easily obtained without using solvents that are harmful to living organisms, and a hollow spherical particle with high stability can be obtained. The solubility is preferably 4 w/v% or more, more preferably 5 w/v% or more, even more preferably 6 w/v% or more, and still more preferably 7 w/v% or more. The upper limit of the solubility is not particularly limited and can be, for example, 40 w/v%, 30 w/v%, or 25 w/v%.

The measurement method for the solubility is as follows. A sample is stirred and dissolved in ethanol at 25°C and the resulting product is separated into an ethanol-soluble part and ethanol-insoluble part by solid-liquid separation. An aqueous ethanol solution at 25°C (1/1 = ethanol/water) is added to the ethanol-insoluble part, and the mixture is stirred for dissolution to obtain an aqueous ethanol solution-soluble part. The aqueous ethanol solution-soluble part is mixed with the ethanol soluble part to obtain a sample solution. The amount of the aqueous ethanol solution added is adjusted so that the ethanol concentration of the sample solution is in the range of 60 to 80%. In the step of obtaining the soluble part, dispersion and solubilization are performed by ultrasonic irradiation, if necessary. The maximum sample concentration at which the sample solution is visually transparent is defined as "solubility in 60 to 80% ethanol at 25°C."

In the lignin-saccharide complex of the present invention, the constituent ratio of saccharide to lignin is preferably 0.01 to 10.

The lignin-saccharide complex of the present invention has a weight average molecular weight (Mw) of preferably 800 to 15,000, more preferably 2,000 to 10,000, and even more preferably 3,000 to 7,000. The lignin-saccharide complex of the present invention has a polydispersity (Mw/Mn) of preferably 3.5 or less, and more preferably 2.5 or less.

The measurement method for each of the above parameters is in accordance with the measurement method for the lignin of the present invention or the measurement method in the Examples described below.

### 3. Hollow Spherical Particle

The hollow spherical particle of the present invention is not particularly limited as long as it is a self-assembled body of the lignin-saccharide complex of the present invention and has a hollow spherical shape. Self-assembling occurs easily, and the conditions for inducing self-assembling can be appropriately set. For example, the lignin-saccharide complex of the present invention can be dispersed in an aqueous solution and self-assembled to form a hollow spherical particle.

Specifically, in the above, self-assembling can be induced by gradually adding water to a sample dissolved in an organic solvent (e.g., alcohol) system to form a hollow spherical particle. The final water content is about 95%, but once capsules are formed, they become stable in water. Alternatively, capsules can be formed by dissolving a sample in alcohol-containing water etc., followed by natural evaporation. This results in the formation of capsules of sub-micron order with high uniformity.

Conventional lignin (commercially available industrial craft lignin) does not exhibit dispersibility or solubility in hydrophilic solvents, such as ethanol, and water-mixed solvents. The lignin-saccharide complex of the present invention exhibits dispersibility and solubility in hydrophilic solvents and water-mixed solvents. Therefore, the use of the lignin-saccharide complex of the present invention results in a hollow spherical particle that is safer for living organisms.

The hollow spherical particle of the present invention contains hollow portions, and the number of hollow portions is not particularly limited. The hollow spherical particle of the present invention can be a mononuclear type with one hollow portion, and can also be a polynuclear type with a plurality of hollow portions.

The self-assembled body of the lignin-saccharide complex of the present invention exhibits fluorescence in the long-wavelength region. Specifically, the self-assembled body of the lignin-saccharide complex of the present invention exhibits fluorescence in the wavelength range of 400 nm to 700 nm. Further, in the self-assembled body of the lignin of the present invention, in particular, fluorescence shifted to significantly shorter wavelength compared with the excitation wavelength, i.e., anti-Stokes fluorescence, is observed. Anti-Stokes fluorescence is observed even with an excitation light source weaker than a conventional light source, such as a xenon lamp. For this reason, the hollow spherical particle of the present invention can be used as an anti-Stokes fluorescent agent. In this case, for example, the hollow spherical particle of the present invention can be used as an anti-Stokes fluorescent paint, ink, and identifier, as well as an upconversion/wavelength conversion molecular material.

The hollow spherical particle of the present invention is stably dispersed in an aqueous solution, and further can maintain the above fluorescence even in a dried solid state.

The hollow spherical particle of the present invention has a number average particle size of 100 µm or less, for example. In a preferred embodiment of the present invention, the hollow spherical particle of the present invention has a number average particle size of 10 nm or more, for example. The number average particle size is preferably 30 nm to 30 µm, more preferably 50 nm to 10 µm, even more preferably 60 nm to 1 µm, still even more preferably 70 nm to 400 nm, and particularly preferably 80 nm to 300 nm.

The hollow spherical particle of the present invention can have a high void ratio. The void ratio of the hollow spherical particle of the present invention is, for example, 60% or more, preferably 70% or more, more preferably 75% or more, and even more preferably 80% or more. The void ratio is measured according to the method described in Reference Test Example 6.

The hollow spherical particle of the present invention can be used, for example, as a carrier for transporting substances by incorporating an active ingredient in their hollow portions. This also makes it possible to impart solubility resistance, compatibility, light resistance, and the like to the active ingredient. The active ingredient can be selected from a wide range. Examples include agricultural chemicals, fertilizers, pharmaceuticals, cosmetic ingredients, fragrances, fluorescent agents, paints (e.g., vanillin, limonene, and menthol), proteins, antibodies, nucleic acids (e.g., oligonucleotides such as DNA and RNA), vaccines, peptides, lipids, hormones, sugars, glycosides, surfactants, ultraviolet absorbers, light-scattering agents, adhesives, vitamins, coenzymes, minerals, cells, organelles, low-molecular-weight compounds (e.g., signaling substances such as pheromones; nitrogen-containing compounds such as alkaloids, monoterpenes, and sesquiterpenes, and isoprenoids such as terpenoids; and phenylpropanoids), high-molecular-weight compounds, metal complexes, liposomes, hydrogels, and the like.

Specifically, the hollow spherical particle of the present invention can be used as a drug carrier (for DDS). More specifically, the hollow spherical particle of the present invention can be used as organic capsules as drug carriers. Since they are natural products and are expected to have biocompatibility, they are useful as drug carriers. Furthermore, hollow spherical particles with an average particle size of 200 nm or less, preferably 100 nm or less, are expected to have an enhanced permeation and retention (EPR) effect that allows them to reach and accumulate in tumor tissue without leaking into normal tissue.

The hollow spherical particle of the present invention can also be used as ultraviolet-absorbing capsules for the purpose of imparting light resistance to protect the encapsulated compound from ultraviolet rays. Since the absorption range includes UV-A and UV-B wavelength ranges, the hollow spherical particle of the present invention is useful as ultraviolet-absorbing materials for use in skin care products such as sunscreens. Such an ultraviolet absorption effect is presumed to be due to intermolecular interactions such as π-π stacking of aromatic lignin.

The hollow spherical particle of the present invention also contains cationic particles, has a high affinity for negatively charged cell membranes, and is easily taken up into cells. Accordingly, it is useful as a drug carrier and can be used as a drug delivery or introducing agent. Further, the hollow spherical particle of the present invention is useful as a nucleic acid carrier and can be used as a nucleic acid delivery or introducing agent because it can form an ionic complex with a negatively charged nucleic acid etc. on its surface. The hollow spherical particle of the present invention can have a positive charge, for example, a zeta potential of 5 to 100 mV, preferably 10 to 80 mV, more preferably 20 to 60 mV, and even more preferably 40 to 60 mV.

In one embodiment of the present invention, the hollow spherical particle of the present invention is a population of hollow spherical particles composed of a lignin-saccharide complex and having an average particle size of 200 nm or more, preferably 250 nm or more, and more preferably 300 nm or more, and has a positive average zeta potential at a pH of 7 and room temperature. Further, in one embodiment of the present invention, the hollow spherical particle of the present invention is a population of hollow spherical particles composed of a lignin-saccharide complex, and having an average particle size of 200 nm or less, and preferably 150 nm or less, and has a negative average zeta potential at a pH of 7 and room temperature. The zeta potential can be measured by the electrical resistance nanopulse method. Room temperature is a temperature around 20°C, and is, for example, 20°C.

The hollow spherical particle of the present invention is positively charged at neutral to acidic pH and can be stably dispersed in water for a long period of time. As shown in Example 3, it functions as a pH-responsive nanocarrier, which can achieve switching from a positive charge to a negative charge with almost no change in the particle size by making the external environmental pH basic.

In the hollow spherical particle of the present invention, a liquid, a gas, or a solid (metal as a type) may be enclosed therein. In this case, the hollow spherical particle of the present invention can be used, for example, as a heat-insulating material, a compatibilizer, a catalyst, or a sensitizer.

The state of presence of the active ingredient, solid, etc. is not particularly limited as long as they are held in contact with the hollow spherical particle. For example, they may be present in the hollow portions of the hollow spherical particle, held by or attached to the constituent component (lignin) of the hollow spherical particle, or held by or attached to the outer surface of the hollow spherical particle.

The hollow spherical particle of the present invention exhibits long-wavelength fluorescence. This is due to the formation of intermolecular and intrapolymer associations of conjugated systems caused by the self-assembling of lignin, known as J-associates, and results in long-wavelength fluorescence due to exciplex formation, preferably fluorescence at 450 nm or more, more preferably fluorescence at 480 nm or more, and even more preferably fluorescence at 500 to 600 nm (specifically, e.g., Ex 515 nm/Em 535 nm), and large Stokes shift fluorescence of 100 nm or more (e.g., Ex 410 nm/Em 670 nm). Fluorescence in the long wavelength region is useful for identifying hollow spherical particles. In particular, fluorescence in the near-infrared region of 600 nm or more is called the "biological window," and is suitable for selective optical detection without overlap with coexisting components, as well as tracking and imaging of drug dynamics in vivo.

The hollow spherical particle, which is a self-assembled body of the lignin-saccharide complex of the present invention, is stable over time, association and aggregation are suppressed, and the particle size can be maintained more stably. The stability (dispersion retention ability, structural stability, and temperature stability) of the hollow spherical particle can be evaluated according to the rate of change in average particle size 6 weeks after production when measured under the conditions of Example 2. Regarding the stability (rate of change in average particle size) of the hollow spherical particle, the rate of change after 6 weeks is within ±13% compared to 100% of the average particle size immediately after production. In particular, the hollow spherical particle has excellent temperature stability, and the rate of change in average particle size is within ±20% (in the range of 10°C to 60°C), and preferably within ±5% (in the range of 10°C to 70°C). Therefore, regarding the stability (6 weeks) of the hollow spherical particle of the present invention, the rate of change is within 15%, and preferably the thermal stability is within 5%.

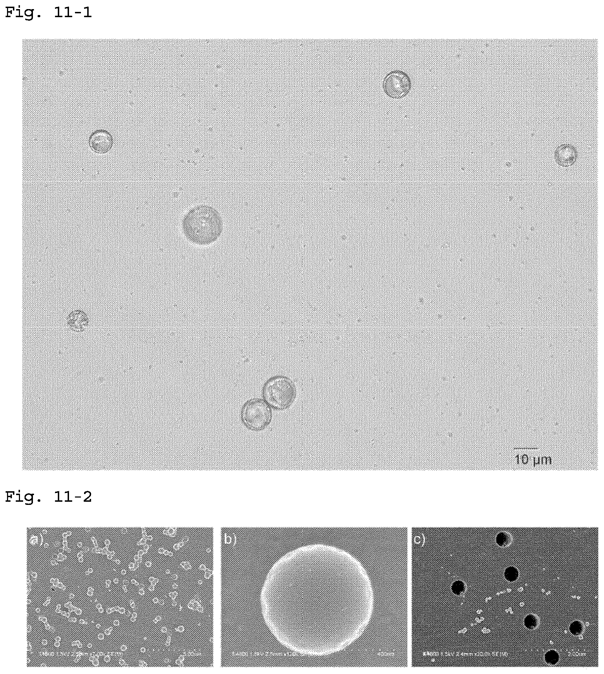

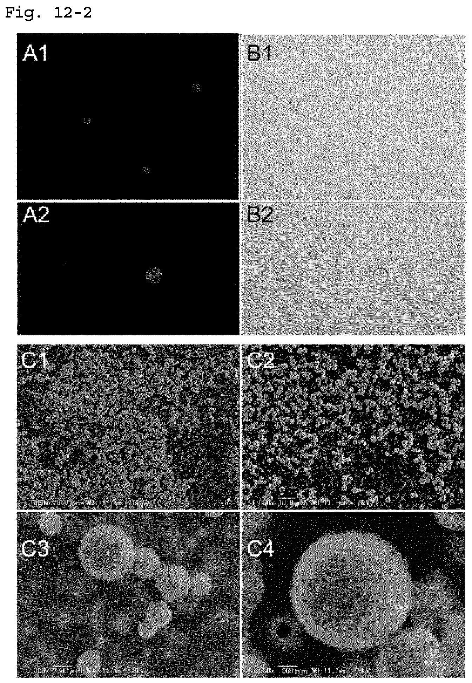

The structural stability can also be evaluated by scanning electron microscopy. That is, as shown in Example 1 (Fig. 11-2), the hollow spherical structure of dried capsules is maintained even in a vacuum. Similarly, after drug encapsulation, the structure remains stable, as shown in Example 1 (Fig. 12-2). Such shape stability is suitable for efficient formulation, accumulation, transportation, and storage of drug carriers.

The particle size, surface charge, dispersibility, and active ingredient encapsulation capacity of the lignin-saccharide complex capsule, which is the hollow spherical particle of the present invention, can be widely applied depending on the plant species and part of the biomass raw material.

This difference is derived from the structural unit ratio of lignin (the constituent ratio of the aromatic nuclear structure based on phenylpropane units, guaiacyl lignin, syringyl lignin, p-hydroxyphenyl lignin, and flavonoid lignin) and the type of polysaccharide in the constituent lignin-saccharide complex.

Furthermore, the particle size, surface charge, dispersibility, and active ingredient encapsulation ability of the lignin-saccharide complex capsule, which is the hollow spherical particle of the present invention, can be widely applied by separating the lignin-saccharide constituent ratio (the ratio obtained by dividing the number of monomer units of polysaccharide by the number of monomer units of lignin) and the type of polysaccharide in the constituent lignin-saccharide complex.

Lignin-saccharide complexes with different lignin-saccharide constituent ratios can be easily separated based on the difference in their solubility in alcohol/water or dioxane/water. They can also be separated by hydrophobic interaction chromatography as described in the following document: Nishimura et al., Direct evidence for α ether linkage between lignin and carbohydrates in wood cell walls, Sci Rep 2018, Toyopearl HW50 column.

The polysaccharide component in the lignin-saccharide complex is hemicellulose. Hardwood raw materials contain a lot of acidic polysaccharides (glucuronoxylan) containing uronic acid, while softwood raw materials contain a lot of neutral polysaccharides (glucomannan). Separation focusing on the polysaccharide components of the lignin-saccharide complex can be achieved using ion exchange chromatography as described in the following document: Nishimura et al., Direct evidence for α ether linkage between lignin and carbohydrates in wood cell walls, Sci Rep 2018.

The sustained release properties of the hollow spherical particle of the present invention can be controlled by the action of an enzyme, which is a biocatalyst. As shown in Example 4, a polysaccharide-degrading enzyme can be applied to deform or disintegrate hollow spherical fine particles carrying a drug, whereby the drug can be released. Enzymes have substrate specificity and pH-selective and temperature-selective activity, and it is possible to control the sustained release conditions (pH, temperature, and release rate) by using enzymes (hydrolases and oxidases) appropriate for the purpose.

The encapsulation and coating of active ingredients, such as metal nanoparticles, using the lignin-saccharide complex of the present invention makes it possible to improve a wide range of functionality. It provides improved particle dispersibility and persistence in water, good dispersibility of dried particles, and compatibility with the matrix containing resin etc. These allow the use of metal nanoparticles in a wide variety of environments and provide hydrophobicity in organic solvents, thereby enhancing solubility and dispersibility. These characteristics are desired in many industrial applications. These coatings also offer the ability to preserve the chemical stability of metal nanoparticles or metal ions and control their reactivity. This enables these particles to function as catalysts or sensors. By improving biocompatibility, environmental affinity, photoresponsiveness, and molecular recognition ability, biomedical applications, environmental remediation, optical devices, biosensors, and other applications are possible. By imparting sustained release properties, it is possible to control the release of substances or the expression of functions over time, which is particularly useful for drug delivery applications. By improving weather resistance and light resistance, the durability of encapsulated components and metal ions against the external environment is improved. Furthermore, controlling the particle size contributes to improving safety, optimizing performance, and improving environmental compatibility and biocompatibility.

Specifically, the present invention brings about the improvement, modification, expanded applications, and performance enhancement of the following metal nanoparticles.

Gold (Au) nanoparticles have applications in the biomedical field, including sensors utilizing surface plasmon resonance (SPR), imaging, carriers in drug delivery systems (DDS), target selectivity, and photothermal therapy using their photothermal conversion properties upon light irradiation. They are also expected to have a wide range of applications as catalysts for chemical reactions, such as organic synthesis reactions, electrode materials for fuel cells, and air and water purification. Silver (Ag) nanoparticles have applications as antibacterial agents taking advantage of their antibacterial properties, and as conductive ink. Platinum (Pt) nanoparticles are widely used, particularly as catalysts in fuel cells, and are also utilized as catalysts in the chemical industry. Palladium (Pd) nanoparticles are particularly useful as catalysts, are used in carbon-carbon bond-forming reactions and hydrogenation reactions, and are promising hydrogen storage materials. Iron (Fe) nanoparticles are expected to be used as contrast agents for MRI and as components of magnetic fluids, and to decompose hazardous substances in groundwater. Copper (Cu) nanoparticles are expected to be used as conductive ink and as catalysts for the decomposition of air pollutants.

Nickel (Ni) nanoparticles are expected to be applied to data storage, magnetic ink, magnetic fluids, etc., taking advantage of their strong magnetic properties. They are also expected to be used as catalysts in hydrogenation reactions and energy conversion. Titanium (Ti) nanoparticles are used, in the form of titanium dioxide (TiO2), as photocatalysts, and as self-purifying materials, and have water/air purification function. They are also expected to be used in bioimaging and drug delivery. Zinc (Zn) nanoparticles are expected to be used in solar power generation, LEDs, UV inhibitors, cosmetics, etc. Aluminum (Al) nanoparticles also expected to be applied to energy generation (e.g., rocket propellants) and as thermal conductors.

### Examples

The present invention will be described in detail below based on Examples. However, the present invention is not limited to these Examples.

### Reference Test Example 1: Isolation of Lignin, Lignin-Saccharide Complex, Hemicellulose, Cellulose-Hemicellulose Complex, and Cellulose

Lignin, lignin-saccharide complexes, hemicelluloses, cellulose-hemicellulose complexes, and cellulose were isolated from biomass according to an M-APA method, an A-APA method, or these methods with altered conditions. The yield, the physical properties, etc. of the isolated products were measured. The isolation methods according to the present invention are capable of isolating soluble lignin, lignin-saccharide complexes, hemicelluloses, cellulose-hemicellulose complexes, and cellulose from natural polymers in biomass with high efficiency, and includes step (A) of bringing a solution containing an organic acid and a peracid into contact with plant biomass. Specifically, the isolation was performed as described below.

### Reference Test Example 1-1: Preparation of Finely Milled Wood Flour

A fine powder (finely milled wood flour) with a volume average particle size of 10 µm (1 to 100 µm) was prepared from wood flour of hardwood (*eucalyptus* or *Fagus crenata*), softwood (*Pinus densiflora, Cryptomeria japonica,* or *Chamaecyparis obtusa*), or a herbaceous plant (Bagasse (sugarcane) or bamboo) by using a ball mill or bead mill method.

An example of preparation methods using a ball mill is described below. A planetary ball mill (P-6, Fritsch GmbH) was used. 2 g of wood flour and 100 g of zirconia beads with a diameter of 3 mm were placed in an 80-cc agate container and sealed in two overpots. After the overpots were vacuumed for 20 minutes, nitrogen gas (inert gas) was injected. A cycle composed of milling for 1 minute and a pause for 1 minute and 20 seconds was performed 180 times at 550 rpm (3 hours in total). The operation under the above conditions provides a fine wood flour while limiting oxidation during milling and denaturation due to heating.

### Reference Test Example 1-2: Preparation of Wood Flour

1 g of wood flour or wood chips (10 mm × 10 mm × 1 mm) of hardwood (*eucalyptus* or *Fagus crenata*), softwood (*Pinus densiflora, Cryptomeria japonica,* or *Chamaecyparis obtusa*), or a herbaceous plant (Bagasse (sugarcane) or bamboo) was placed in the left and right two pots of a mixer mill (MM301, Retsch). Under the conditions of a grinding time of 5 minutes and a frequency of 1/15 sec, milling was performed, thereby obtaining a powder (wood flour) with a volume average particle size of 0.1 mm.

### Reference Test Example 1-3: M-APA Method (Milling-Acid-PerAcid Method)

For a starting material, the finely milled wood flour (average particle size: 10 µm) obtained in Reference Test Example 1-1 was used. 2 g of finely milled wood flour and 10 mL of a solution containing acetic acid and peracetic acid (composition: 17.4M acetic acid 99%, 0.14M peracetic acid less than 1%, 0.01M hydrogen peroxide, and a trace amount of water) were placed in a vial for the Initiator and irradiated with microwaves for 10 minutes at 50°C, initial power of 400 W, and stirring speed of 600 rpm with an Initiator+ 60 microwave synthesizer (Biotage). After the soluble part was treated with microwaves, 10 mL of a separating solvent (acetone) was added, followed by separation by spin-down (RCF 8000 × g, 5 min). The insoluble part was extracted with 10 mL of a separating solvent (acetone) and washed three times, and combined with the soluble part. This soluble lignin is referred to as acid peracid lignin (APA lignin).

The insoluble part was subjected to the same extraction operation with the microwave heating condition changed to 140°C as the second step. The soluble part is referred to as a lignin-hemicellulose complex. An insoluble part containing cellulose as a main component was obtained.

### Reference Test Example 1-4: A-APA Method (Alkali-Acid-PerAcid Method)

For a starting material, the wood flour (average particle size: 0.1 mm) obtained in Reference Test Example 1-2 was used. A 0.25M sodium hydroxide solution was prepared (1N diluted 4 times). 2.4 mL of the 0.25 M sodium hydroxide solution was added to 0.3 g of wood flour, followed by microwave extraction treatment (100°C, 30 min). A rough indication of the required amount of an alkali is 1 mmol of NaOH per gram of wood flour.

After microwave treatment, the soluble part and the insoluble part can be easily separated by decantation, spin-down, or filtration. Extraction with acetone (separating solvent) was additionally performed three times, and the extract was combined with the soluble part. From the insoluble part, acid peracid lignin (APA lignin) and a lignin-hemicellulose complex were obtained as soluble lignin according to the same extraction method as the M-APA method of Reference Test Example 1-3. An insoluble part containing cellulose as a main component was obtained.

### Reference Test Example 1-5: Study of Conditions

Instead of acetic acid, other organic acids (formic acid, glyoxylic acid) were used for the study. Additionally, another peracid (hydrogen peroxide) was used instead of peracetic acid. For another peracid (hydrogen peroxide), the composition was changed as follows: acetic acid 12M, and hydrogen peroxide concentrations shown in the following table.

The study was conducted by changing the temperature at which plant biomass or an insoluble part comes in contact with an organic acid and a peracid to a temperature ranging from room temperature to 180°C. Additionally, at this time, the heating method was also changed from the internal heating method using microwave heating to an external heating method using an oil bath in conducting the study.

### Reference Test Example 1-6: Analysis and Measurement Method

Lignin, lignin-saccharide complexes, and cellulose were analyzed and measured according to the following methods.

### Reference Test Example 1-6-1: Measurement Method for Molecular Weight (Mn, Mw, and Degree of Polydispersity)

The molecular weight was determined by size exclusion chromatography (SEC), i.e., gel permeation chromatography (GPC). A sample was dissolved in a solution of acetic anhydride and a base (e.g., pyridine) and reacted for 3 to 6 hours to obtain an acetylated product. The acetylated product was dissolved in tetrahydrofuran (THF) to a concentration of 2.0 mg/mL, and separation was performed by high-performance liquid chromatography (high-pressure gradient SEC analysis system manufactured by Shimadzu Corporation). The columns for use were HZ-M columns (Tosoh Corporation, 150 mm × 4.6 mm id, 4 µm, three columns connected in series). The analysis was performed by injecting 10 µL of the sample at a column temperature of 40°C using tetrahydrofuran (THF) as the mobile phase at a flow rate of 0.35 mL/min. A mass calibration curve was prepared using standard polystyrene, Tosoh PStQuick C (molecular weights (MW): 2,110,000, 427,000, 37,900, and 5970), pinoresinol (MW: 352), and vanillin (MW: 152) to determine the weight average molecular weight Mw and the number average molecular weight Mn of the sample. The degree of polydispersity was determined as Mw/Mn.

### Reference Test Example: 1-6-2: Method for Calculating Constituent Ratio of Binding Units in Molecule

Samples were dissolved in deuterated dimethyl sulfoxide (DMSO-d₆) to 1 to 10 wt%, and their NMR spectra were obtained using a Bruker Avance III 600 MHz NMR Spectrometer equipped with a low-temperature probe. The constituent ratio of the interunit binding units of lignin was calculated from the volume of signals on the spectra of heteronuclear single-quantum correlation spectroscopy (HSQC). The constituent ratio was calculated as a ratio per 100 aromatic rings of lignin. The content of the ether binding unit was estimated from the sum of the integrated values of the C-H correlation signal at the β position (δH/δC = the region of 4.0 ± 0.2 ppm/85.6 ± 1.2 ppm, δH/δC = the region of 4.3 ± 0.2 ppm/83.0 ± 0.7 ppm). The quantitative value in the two-dimensional NMR method was corrected according to the TAF method described in a document (Okamura, H. Nishimura, T. Nagata, T. Kigawa, T. Watanabe, and M. Katahira, Accurate and molecular-size-tolerant NMR quantitation of diverse components in solution, Scientific Reports, 6, 21742, 2016) to calculate the accurate content.

### Reference Test Example 1-6-3: Method for Calculating Constituent Ratio of Lignin and Polysaccharides

The sum of integrated values of the C-H correlation signals at the anomeric position (first position) of polysaccharides was calculated according to the NMR method described above and corrected according to the TAF method, followed by calculating the ratio per 100 aromatic rings of lignin.

### Reference Test Example 1-6-4: Method for Calculating Constituent Ratio of Lignin-Saccharide Copolymerization Point

In the same manner as described above, the sum of integrated values of the C-H correlation signals derived from the binding units of the LCα ether and the LCα ester (LC ether, δH/δC = the region of 4.50 ± 0.15 ppm/80.1 ± 1 ppm; LC ester, δH/δC = the region of 5.88 ± 0.15 ppm/74.0 ± 1.5 ppm) was calculated according to the 2D HSQC NMR method and corrected according to the TAF method, followed by calculating the ratio per 100 aromatic rings of lignin. In the A-APA method, although some of the ester bonds are hydrolyzed by an alkali treatment, an LCα ester-type lignin-hemicellulose complex can also be obtained.

### Reference Test Example: 1-6-5: Method for Calculating Yield from Raw Material Biomass

Each of the obtained components was confirmed with a pH meter, pH test paper, or peracid test paper (potassium iodide starch paper), and then neutralized and subjected to peracid quenching as necessary. Neutralization was performed by adding 1N HCl or 1N NaOH in small amounts stepwise. Peracid quenching was performed by adding a 1M aqueous sodium sulfite solution dropwise. The organic solvent was distilled off by using an evaporator. Subsequently, a dry powder was obtained by lyophilization. When a salt was produced by neutralization, the concentrated solution obtained after distillation of the organic solvent was re-extracted with a separating solvent (acetone) and desalted. Thereafter, the solvent was distilled off again with an evaporator, and lyophilization was performed, thereby obtaining a dry powder. Each of the obtained components was weighed, and the yield per raw material biomass was calculated.

### Results

A polymer with a desirable molecular structure, molecular weight, and yield can be obtained under the following conditions. Tables 1 to 7 show the results.

Table 1 shows the peracid conditions and the yield of each component of polymeric lignocellulose in the two-stage microwave extraction reaction according to the M-APA method (wt%, raw-material biomass ratio, raw material: *eucalyptus*).

**Table 1**

| **Peracid concentration conditions** | **Peracetic acid 1% 0.14 mol/L** | **H₂O₂ 0.4% 0.11 mol/L** | **H₂O₂ 3.2% 1.0 mol/L** | **H₂O₂ 4.2% 1.4 mol/L** |
|---|---|---|---|---|
| **APA lignin** | **7** | **6** | **9** | **11** |
| **Lignin-hemicellulose copolymer** | **12** | **11** | **14** | **25** |
| **Residual cellulose** | **79** | **82** | **75** | **63** |
| **Total yield** | **98** | **98** | **98** | **99** |

Table 2 shows the yield of each component of polymeric lignocellulose of various kinds of biomass in the two-stage microwave extraction reaction according to the A-APA method (wt%, raw-material biomass ratio).

**Table 2**

| | **Hardwood** | | **Softwood** | | | **Herbaceous plant** | |
|---|---|---|---|---|---|---|---|
| **Raw-material biomass plant species** | *Eucalyptus* | *Fagus crenata* | *Pinus densiflora* | *Cryptomeria japonica* | *Chamaecyparis obtusa* | **Bagasse (sugarcane)** | **Bamboo** |
| **Weakly alkaline soluble lignin** | **21** | **22** | **26** | **20** | **19** | **33** | **32** |
| **APAlignin** | **5** | **5** | **4** | **5** | **6** | **4** | **5** |
| **Lignin-hemicellulose copolymer** | **32** | **23** | **14** | **14** | **12** | **12** | **15** |
| **Total soluble part** | **58** | **51** | **44** | **40** | **37** | **50** | **51** |
| **Residual cellulose** | **44** | **39** | **58** | **65** | **61** | **48** | **52** |
| **Total** | **102** | **90** | **103** | **105** | **98** | **98** | **104** |

Table 3 shows the peracid conditions and the molecular weight of each component of polymeric lignocellulose in the two-stage microwave extraction reaction according to the M-APA method (GPC analysis values of acetylated products).

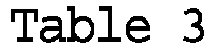

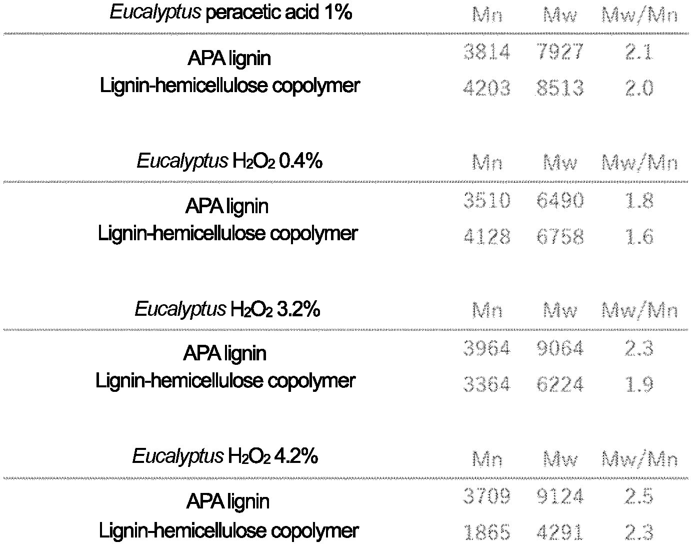

Table 4 shows the yield of each component of polymeric lignocellulose of various kinds of biomass in the two-stage microwave extraction reaction according to the M-APA method (wt%, raw-material biomass ratio).

**Table 4**

| | **Hardwood** | | **Softwood** | | **Herbaceous plant** | |
|---|---|---|---|---|---|---|
| **Biomass species** | *Eucalyptus* | *Fagus crenata* | *Pinus densiflora* | *Cryptomeria japonica* | **Bagasse (sugarcane)** | **Bamboo** |
| **APAlignin** | **7** | **4** | **7** | **4** | **7** | **11** |
| **Lignin-hemicellulose copolymer** | **14** | **9** | **8** | **9** | **13** | **18** |
| **Residual cellulose** | **71** | **84** | **83** | **85** | **79** | **69** |
| **Total yield** | **92** | **97** | **98** | **97** | **99** | **98** |

Solvent: Acetic acid (containing 1% of peracetic acid in an amount of 0.14 mol/L)
Microwave Extraction Conditions: An APA lignin soluble part was obtained (50°C, 10 minutes, first stage); A lignin hemicellulose complex was obtained (140°C, 10 minutes, second stage).

Table 5 shows the yield of each component of polymeric lignocellulose of various kinds of biomass in the first-stage microwave extraction reaction according to the A-APA method (wt%, raw-material biomass ratio).

**Table 5**

| | **Hardwood** | | **Softwood** | | | **Herbaceous plant** | |
|---|---|---|---|---|---|---|---|
| **Raw-material biomass plant species** | *Eucalyptus* | *Fagus crenata* | *Pinus densiflora* | *Cryptomeria japonica* | *Chamaecyparis obtusa* | **Bagasse (sugarcane)** | **Bamboo** |
| **Weakly alkaline soluble lignin** | **22** | **22** | **25** | **19** | **18** | **35** | **32** |
| **APAlignin + lignin polysaccharide complex** | **30** | **26** | **13** | **21** | **19** | **12** | **19** |
| **Total soluble part** | **52** | **48** | **39** | **40** | **37** | **47** | **51** |
| **Residual cellulose** | **54** | **55** | **63** | **65** | **68** | **51** | **55** |
| **Total** | **106** | **103** | **101** | **105** | **105** | **98** | **106** |

Table 6 shows the molecular weight of each component of polymeric lignocellulose of various kinds of biomass obtained according to the A-APA method (GPC analysis values of acetylated products).

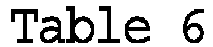

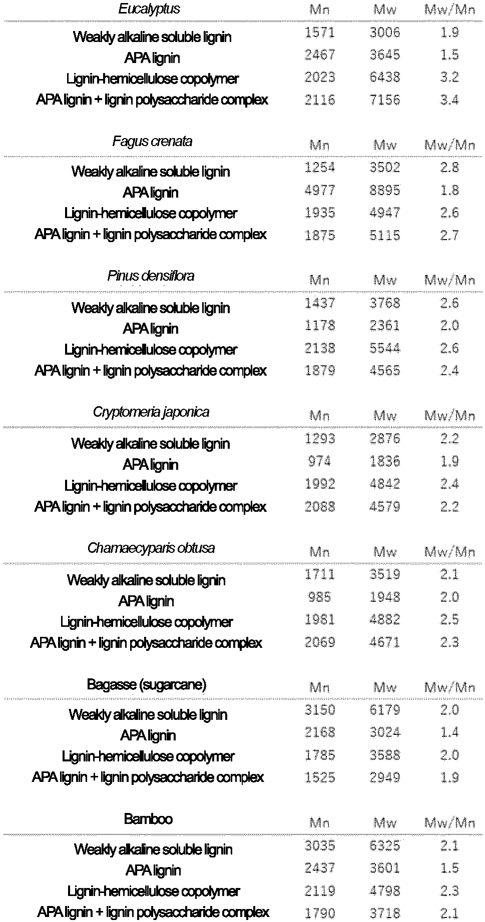

The study of conditions found that when the starting material is wood-based biomass, the temperature suitable for contact with an organic acid and a peracid is 40 to 90°C for obtaining soluble lignin; and that the temperature suitable for contact with an organic acid and a peracid is 100 to 160°C for obtaining a lignin-hemicellulose complex. The study of conditions also found that when the starting material is herbaceous biomass, the temperature suitable for contact with an organic acid and a peracid is from room temperature to 60°C for obtaining soluble lignin; and that the temperature suitable for contact with an organic acid and a peracid is 70 to 150°C for obtaining a lignin-hemicellulose complex.

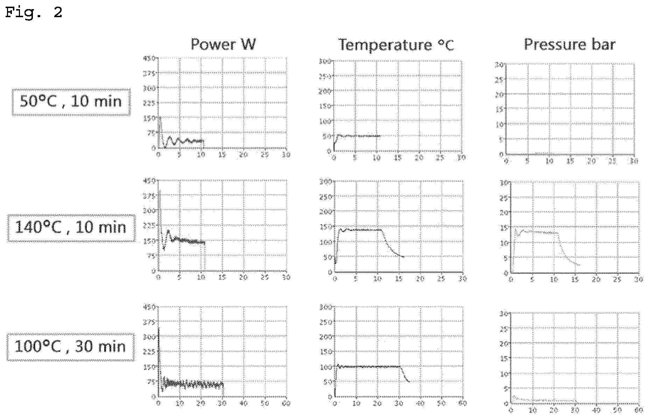

The study of conditions found that the heating time by microwave irradiation may be 5 to 30 minutes, and preferably 10 minutes. The net irradiation time was short. See Fig. 2 for the microwave profile, and data on temperature and pressure.

The study of conditions also found that the microwave heating resulted in a higher yield and a higher selectivity at low temperature in a short time with a low energy input than the external heating method. More specifically, microwave effects such as local heating, internal heating, electron transfer, and a chemical-permeation-promoting effect were observed.

The study of conditions also found that acetic acid, formic acid, and glyoxylic acid (organic acids) were all able to isolate lignin, and that acetic acid was most suitable from the viewpoint of yield, purity, and industrial application.

Fig. 3 shows the results of NMR in Reference Test Examples 1-6-2 and 1-6-3. Fig. 3 shows the results of analysis of lignin isolated from *eucalyptus* (starting material) according to the M-APA method (microwave treatment at 50°C for 10 minutes with acetic acid-peracetic acid 1%). Quantification of the proportion of interunit bonds of lignocellulose according to the TAF-NMR method found that the signal derived from lignin was 99%, and that the signal derived from polysaccharides (hemicelluloses) was 1%. The breakdown of the interunit bonds of lignin was the following: 81% of ether-type β-O-4, 5% of β-5, 11% of β-β, 1% of dibenzodioxocin, and 1% for the remainder. See Fig. 4 for the partial chemical structures of the interunit bonds of lignin and the covalent bonds of lignin-hemicellulose.

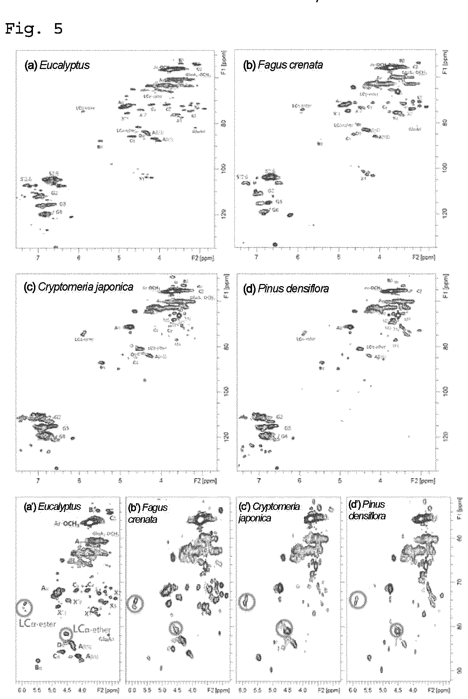

Fig. 5 shows the results of NMR in Reference Test Example 1-6-4. Fig. 5 shows the results of analysis of the lignin-hemicellulose complex isolated from individual plants (starting materials) according to the M-APA method (microwave treatment at 50°C for 10 minutes with acetic acid-peracetic acid 1%). The LC bonds (ether type and ester type) indicated by circles in the figure are a main branched structure present in a ratio equivalent to or higher than the abundance ratio of β-5 and β-β bonds, which are the main interunit bonds of lignin. The isolated lignin-hemicellulose complex is characterized in particular by the presence of an LCα-ester linkage.

### Reference Test Example 2: Solubility Measurement

The lignin and the lignin-hemicellulose complex obtained according to the method of Reference Test Example 1 or a method similar thereto were measured for the solubility in 60 to 80% ethanol at 25°C. Table 7 shows the details of the measurement samples. The abbreviations in Table 7 are as listed below. Similar abbreviations are also used in parts of the specification hereafter.
PA: Peracetic Acid
HP: Hydrogen Peroxide
50: MW50 ACTN, treatment with microwaves at 50°C, high-quality polymeric lignin
140: MW50-Mw140 ACTN, after residues were treated with microwaves at 50°C, the residues were treated at 140°C (second stage), a lignin-hemicellulose complex
A50: An acetic acid-peracetic acid system, HP PA1% AA Mw50 ACN, high-quality polymeric lignin treated with microwaves at 50°C
H50: An acetic acid hydrogen peroxide system, HP 1M hydrogen peroxide, Mw50 ACN, high-quality polymeric lignin treated with microwaves at 50°C

**Table 7**

| | | |
|---|---|---|
| **EA50** | *Eucalyptus* **Chip** | **Acetic Acid Peracetic Acid System PA1%AA Mw50 ACTN** |
| **EA140** | *Eucalyptus* **Chip** | **Acetic Acid Peracetic Acid System PA1%AAMW50-Mw140 ACTN** |
| **EH50** | *Eucalyptus* **Chip** | **Acetic Acid Hydrogen Peroxide System HP 1M Hydrogen Peroxide Mw50 ACN** |
| **BH50** | **Domestic** *Fagus crenata* **Sapwood** | **Acetic Acid Hydrogen Peroxide System HP 1M Hydrogen Peroxide Mw50 ACN** |
| **PH50** | **Domestic** *Pinus densiflora* **Sapwood** | **Acetic Acid Hydrogen Peroxide System HP 1M Hydrogen Peroxide Mw50 ACN** |
| **CH50** | **Domestic** *Cryptomeria japonica* **Sapwood** | **Acetic Acid Hydrogen Peroxide System HP 1M Hydrogen Peroxide Mw50 ACN** |
| **CH140** | **Domestic** *Cryptomeria japonica* **Sapwood** | **Acetic Acid Peracetic Acid System HP 1M Hydrogen Peroxide MW50-Mw140 ACTN** |
| **TA50** | **Domestic Bamboo:** *Phyllostachys edulis* **powder** | **Acetic Acid Peracetic Acid System PA1%AAMw50ACTN** |
| **TA140** | **Domestic Bamboo:** *Phyllostachys edulis* **powder** | **Acetic Acid Peracetic Acid System PA1%AAMW50-Mw140 ACTN** |
| **HP50** | **Domestic Bamboo:** *Phyllostachys edulis* **powder** | **Acetic Acid Hydrogen Peroxide System HP 1M Hydrogen Peroxide Mw50ACN** |

The measurement method for the solubility is as follows. A sample was stirred and dissolved in ethanol at 25°C, and the resulting product was separated into an ethanol-soluble part and ethanol-insoluble part by solid-liquid separation. An aqueous ethanol solution at 25°C (1/1 = ethanol/water) was added to the ethanol-insoluble part, and the mixture was stirred for dissolution to obtain an aqueous ethanol solution-soluble part. The aqueous ethanol solution-soluble part was mixed with the ethanol soluble part to obtain a sample solution. The amount of the aqueous ethanol solution added was adjusted so that the ethanol concentration of the sample solution was in the range of 60 to 80%. In the step of obtaining the soluble part, dispersion and solubilization were performed by ultrasonic irradiation, as necessary. The maximum sample concentration at which the sample solution was visually transparent was defined as "solubility in 60 to 80% ethanol at 25°C."

Table 8 shows the results. The lignin and lignin-hemicellulose complexes obtained by the method of the present invention exhibited a solubility of 8 to 20% in ethanol/aqueous solutions (60/40 and 80/20 v/v%).

**Table 8**

| | **Solubility** |
|---|---|
| **EA50** | **8 w/v% in 80% ethanol aqueous solution** |
| **EA140** | **8 w/v% in 80% ethanol aqueous solution** |
| **EH50** | **15 w/v% in 60% ethanol aqueous solution** |
| **BH50** | **20 w/v% in 60% ethanol aqueous solution** |
| **PH50** | **20 w/v% in 60% ethanol aqueous solution** |
| **CH50** | **8 w/v% in 70% ethanol aqueous solution** |
| **CH140** | **20 w/v% in 60% ethanol aqueous solution** |
| **TA50** | **8 w/v% in 80% ethanol aqueous solution** |
| **TA140** | **8 w/v% in 80% ethanol aqueous solution** |
| **HP50** | **15 w/v% in 60% ethanol aqueous solution** |

### Reference Test Example 4: Preparation and Analysis of Self-assembled Body

A self-assembled body was prepared from lignin (sample) isolated from *eucalyptus* or bamboo (starting material) according to the M-APA method (microwave 50°C, 10 min-treatment, acetic acid-peracetic acid 1% or acetic acid-0.2M hydrogen peroxide) or a lignin-saccharide complex (sample) isolated from *eucalyptus* (starting material) according to the M-APA method (microwave 140°C, 10 min-treatment, acetic acid-peracetic acid 1% or acetic acid-0.2M hydrogen peroxide). Specifically, the preparation was performed as described below.

A DMSO solution with a sample concentration of 1 to 2 wt% was prepared and diluted to 0.01 to 0.02 wt% with a mixed solvent containing a separating solvent (acetonitrile or ethanol) and water (or a buffer) (1:1 or 2:1) to obtain a sample solution. 0.1 to 0.2 mL of the sample solution was applied to a glass slide or a cover glass, and the solvent was evaporated under atmospheric pressure to obtain a self-assembled body in the form of a thin film. Of the above conditions, when an equal amount of water (or a buffer) was added to a DMSO solution with a sample concentration of 1 wt%, and the solution was applied, a highly concentrated self-assembled body was obtained.

Subsequently, analysis was performed on the optical characteristics of the obtained self-assembled body (a solid thin lignin film or a solid thin lignin-saccharide complex film) and a comparative object (lignin obtained according to the APA method (APA lignin)). Specifically, the analysis was performed as follows.

The measurements were performed with the following.

UV-visible Absorption Spectrum: UV-2700 (manufactured by Shimadzu Corporation)
Fluorescence Spectrum: Spectrofluorometer RF-6000 (manufactured by Shimadzu Corporation)
Fluorescence Microscope Measurement: All-in-One Fluorescence Microscope BZ-X810 (manufactured by Keyence Corporation)

Analysis was performed with accompanying analysis software of the devices.

UV-visible Absorption Spectrum: Black Quartz Cell for
Spectroscopic Measurement (optical path length: 10 mm)
Fluorescence Measurement: a fully transparent synthetic quartz
cell (optical path length: 10 mm) and a Starna triangular quartz cell SF3 were used.

A solid measurement unit was used for the solid sample. The sample applied to a cover glass was sandwiched and immobilized with quartz plates to perform measurement.

3D fluorescence spectra were measured at an excitation wavelength of 310 to 800 nm, a data interval of 5 nm, a fluorescence wavelength of 350 to 900 nm, a data interval of 1 nm, and a scan speed of 12000 nm/min, with an ultraviolet cut filter U310 mounted on the excitation side in order to reduce the influence of scattered light and multidimensional light. The bandwidth was 5 nm on the excitation side, and 15 nm on the fluorescence side, with the sensitivity set to low.

The synchro-scanning spectrum is a scan mode in which the excitation spectrometer and the fluorescence spectrometer of a spectrofluorometer are simultaneously allowed to perform scanning with these spectrometers shifted from each other by a predetermined wavelength interval. The measurement was performed by varying the wavelength interval between excitation light and fluorescence within the range of -90 nm to 260 nm. A fluorescence having a wavelength shorter than that of excitation light (i.e., a negative wavelength interval) is detected. At this time, a fluorescence caused by an anti-Stokes shift can be selectively detected. The scanning was set so as to cover the fluorescence wavelength range of 350 to 800 nm, with the data interval set to 0.1 nm, the scanning speed to 600 nm/min, the bandwidth to 15 nm on the excitation side and 20 nm on the fluorescence side, and the sensitivity to low.

The UV-visible absorption spectra were measured with a double monochromator UV-2700. Wavelength scanning (200 to 900 nm) was performed.

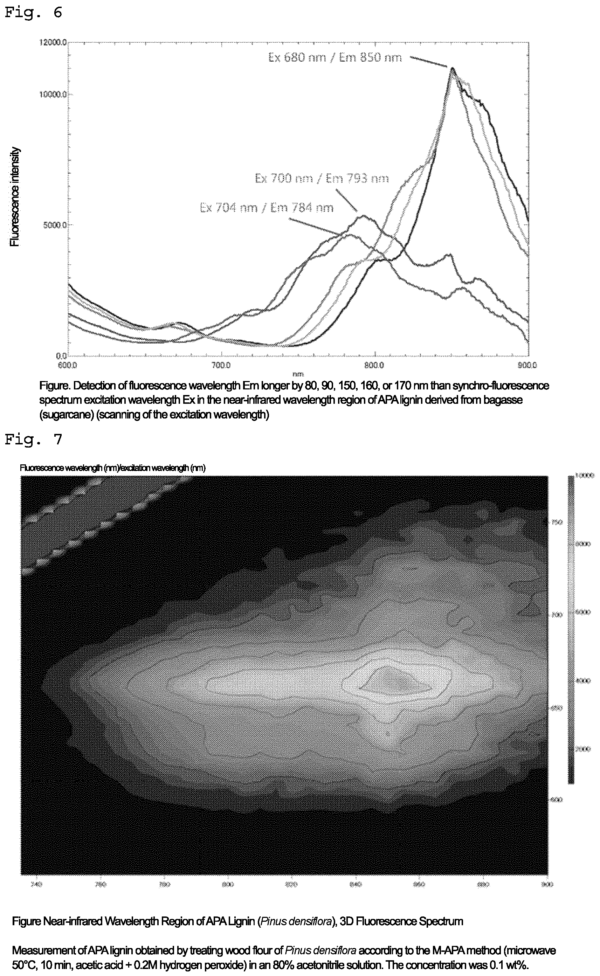

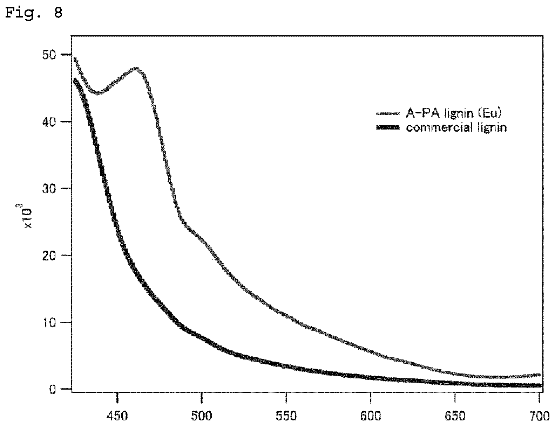

Figs. 6 to 9 show the results. From Fig. 9, the lignin self-assembled body was found to exhibit anti-Stokes fluorescence.

In addition, a complex with gold was synthesized using the obtained self-assembled body (hollow spherical particle of the lignin-saccharide complex). An aqueous solution containing a self-assembled body solution and tetrachloroauric(III) acid (FUJIFILM Wako Pure Chemical Corporation, special grade) was prepared, the atmosphere in the reaction vessel was replaced with argon gas, and then the reaction vessel was sealed. The solution was irradiated with 28 kHz ultrasonic waves for 10 minutes (Three-Frequency Ultrasonic Cleaner VS-10III, produced by Honda Electronics Co., Ltd. (AS ONE)). The solution was filtered through a filter (pore size: 1 µm, produced by pluriSelect Life Science), and the filtrate was analyzed using a dynamic light scattering meter (SZ-100 Nanoparticle Size Analyzer, produced by Horiba Ltd.) and a UV-visible spectrophotometer (UV-2700, produced by Shimadzu Corporation).

As a result, the particle size distribution of the self-assembled body measured with the dynamic light scattering meter showed that the particle size of the gold complex (Au complex 5003, solid line) was smaller than that of the self-assembled body alone (LCC4936, dashed line). The mode diameters were 143.8 nm and 113.5 nm, respectively.

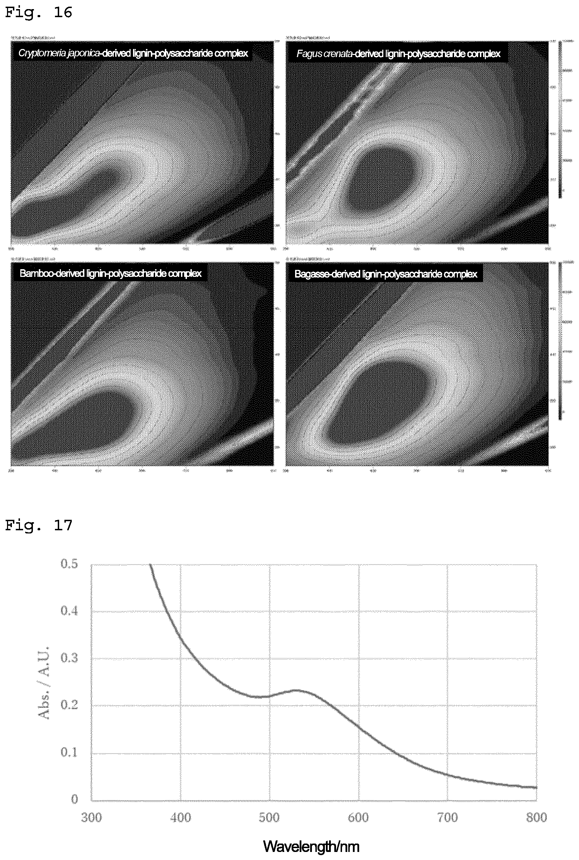

Fig. 17 shows an example of the UV-visible absorption spectrum of the solution after stirring. The absorption observed at 530 to 540 nm can be attributed to the surface plasmon resonance absorption of gold (Creighton et al., J. Chem. Soc. Faraday Trans., Vol. 87, p. 3881, 1991), which indicates that gold ions are reduced by reaction with the self-assembled body to produce stable gold nanoparticles.

Unreduced gold(III) ions in the reaction solution can be quantified as follows (Mizukoshi et al., Analytical Chemistry, Vol. 45, No. 4, p. 327, 1996). A predetermined amount of an aqueous solution containing a self-assembled body solution and tetrachlorogold(III) was reacted for a predetermined time. A saturated aqueous solution of sodium bromide was added to the reaction solution, and aggregates formed by salting-out were separated by filtration, centrifugation, etc. This eliminated the spectral interference caused by the nanoparticles. On the other hand, unreacted gold ions coordinated with the added bromide ions, and the absorption of tetrabromogold(III) ions appeared at around 381 nm. Using a previously prepared calibration curve, unreacted gold ions in the reaction solution were determined colorimetrically. As a result, the amount of gold ions reduced and electrons donated to the gold ions during reduction were calculated, and the amount of electrons donated per mg of the self-assembled body was determined.

For example, 4 µmol of gold ions were consumed by 1.9 mg of the self-assembled body. This indicates that 4 × 3 µmol of electrons were donated to the gold ions. That is, 6.3 µmol of electrons can be donated per mg of the self-assembled body.

### Reference Test Example 4: Measurement of UV Absorption

The UV absorption properties of the lignin and the lignin-hemicellulose complexes used in Reference Test Example 2 were measured. Specifically, the samples were individually dissolved in ethanol to 0.005 w/v%, and the UV absorption spectra of the obtained (transparent) solutions were measured with a spectrophotometer (UV-2700 manufactured by Shimadzu Corporation, optical path length: 1 cm, quartz cell).

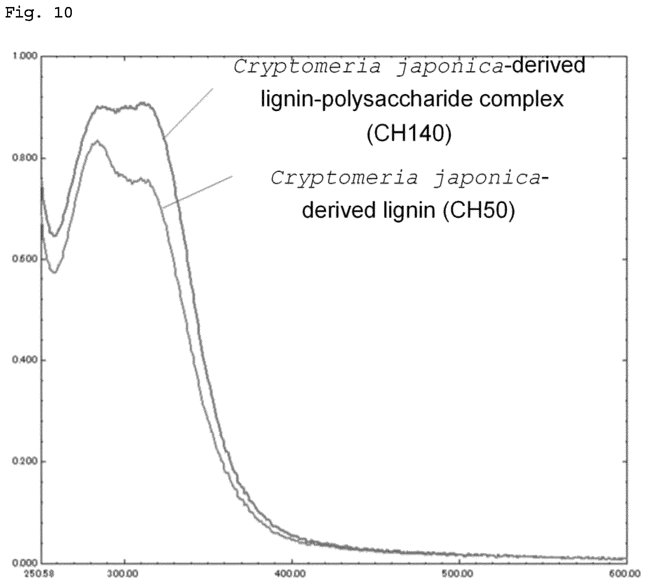

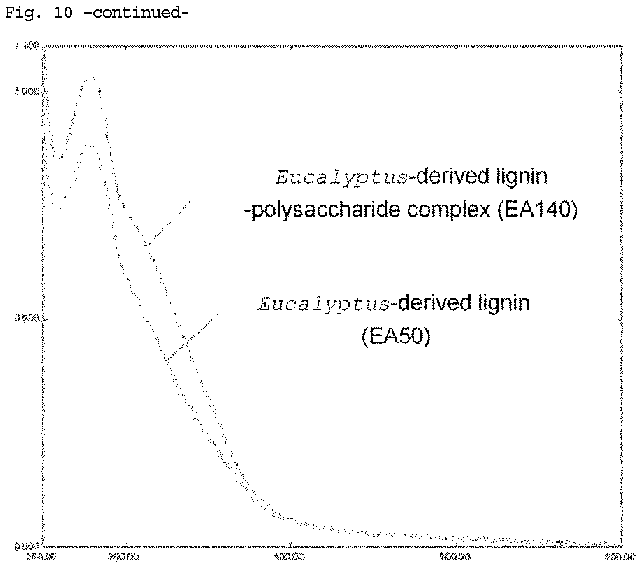

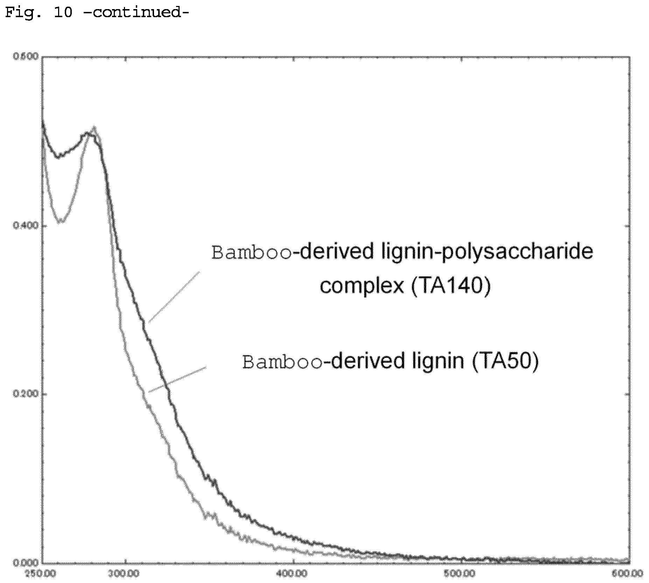

Fig. 10 shows the results. The results indicate that the lignin and lignin-hemicellulose complexes obtained by the method of the present invention exhibited a wide range of UV absorption properties over the UV-A (320 nm to 400 nm) and UV-B (280 nm to 320 nm) wavelength ranges.

### Reference Test Example 5. Preparation of Hollow Spherical Particles 1

The lignin used in Reference Test Example 2 was used as a sample to prepare hollow spherical particles, which are a self-assembled body. Specifically, the preparation was performed as described below.

(1) 20 mg of a lignin sample (dry powder) was dissolved in 1.0 mL of DMSO to prepare a 2% w/v lignin solution.
(2) 0.1 mL of 2% w/v lignin stock solution was added to 0.9 mL of 80% v/v ethanol solution, and the mixture was stirred and mixed uniformly.
(3) 3 mL of water was gradually added dropwise while stirring to dilute the mixture. The dilution rate can be changed. The formation of hollow spherical particles began at an organic solvent concentration of 40% or less, and a stable dispersion state was observed at 5 to 30%.

Alternatively, hollow spherical particles were obtained by a method employing the following step (4) instead of step (3).

(4) 1 mL of an alcohol-water solvent was slowly added dropwise while stirring. The alcohol-water solvent used was a 30% ethanol solution or a 10% n-butanol solution. Thereafter, the solvent was distilled off by gently spraying the solution surface with nitrogen gas, or by drying in air for evaporation, or under reduced pressure. The formation of hollow spherical particles began at an organic solvent concentration of 40% or less, and a stable dispersion state was observed at 5 to 30%.

Fig. 11-1 shows a bright-field image of the resulting hollow spherical particles. Relatively large particles (particle size: 5 µm or more) and relatively small particles (particle size: less than 5 µm) were confirmed.

In addition, the particle size and film thickness of one relatively large particle were measured, and the volume occupied by the cavity of the hollow spherical particle (void ratio) was calculated. Specifically, Keyence All-in-One Fluorescence Microscope BZ-X810 was used to capture fluorescence images using a 100x objective lens in sectioning mode, and the particle size and film thickness were measured from the resulting image data using Keyence analysis software. The void ratio (unit: %) was calculated by the formula: void ratio = (volume of cavity portion of hollow spherical particle (excluding film portion)/total volume of hollow spherical particle) × 100. As a result, the particle size was 21.1 µm, the film thickness was 635 nm, and the void ratio was 83%.

### Reference Test Example 6. Preparation of Hollow Spherical Particles 2

The lignin used in Reference Test Example 2 was used as a sample to prepare hollow spherical particles encapsulating a drug. Specifically, the preparation was performed as described below.

A 0.2% w/v lignin solution (in 80% ethanol) was mixed with 1.0 mL of 0.1% w/v near-infrared fluorescent dye DiR (DiIC18(7)) in 80% ethanol solution while stirring. Water or an alcohol-water solvent was added dropwise with stirring and mixed to reduce the organic solvent concentration in the same manner as in Reference Test Example 5, thereby forming hollow spherical particles.

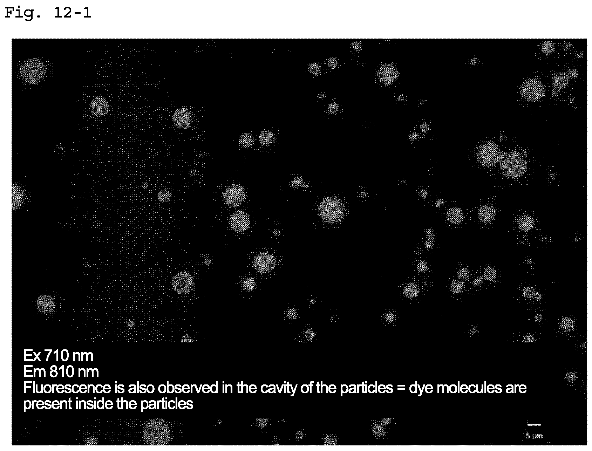

Fig. 12-1 shows an image observed by a fluorescent microscope. Particles exhibiting near-infrared fluorescence derived from the encapsulated dye were observed.

### Reference Test Example 7. Preparation of Hollow Spherical Particles 3

The lignin used in Reference Test Example 2 was used as a sample to prepare hollow spherical particles. Specifically, the preparation was performed as described below.

A 2.5 wt% solution (30% ethanol) of lignin (derived from *eucalyptus*) was prepared and stirred to obtain a uniform solution, which was used as the stock solution. To 100 µL of the stock solution, 200 µL of a 50% organic solvent (ethanol, 1-propanol, allyl alcohol, 2-propanol, or 1-butanol) was gradually added with stirring to obtain a uniform solution. 1.7 mL of water was gradually added with stirring to obtain a uniform solution. The resulting liquid (final concentration 0.125 wt%, organic solvent 5% (containing 0.015% ethanol)) was subjected to nanoparticle measurement by DLS. For DLS measurement, 2 mL of a sample was placed in a disposable glass cell using a multi-sample nanoparticle size measurement system, and the particle size was measured by the dynamic light scattering method. The measurements were performed three times for each, and the average value was calculated. The measurement temperature was 25°C, and the solvent refractive index was 1.33.

The results are shown in Table 9.

**Table 9**

| **Organic solvent** | **Average particle size [nm] (Cumulant)** | **Polydispersity index** |
|---|---|---|
| **Ethanol** | **146.5** | **0.122** |
| **1-Propanol** | **89.2** | **0.163** |
| **Allyl alcohol** | **89.1** | **0.138** |
| **2-Propanol** | **94.5** | **0.166** |
| **1-Butanol** | **214.6** | **0.219** |

In addition, the same tests as above were conducted using lignin derived from other tree species (*Pinus densiflora, Chamaecyparis obtusa, Cryptomeria japonica, Fagus crenata,* and bamboo) (lignin obtained by the same method as in Reference Test Example 2), and similar results were obtained for the average particle size and polydispersity index.

### Example 1. Preparation of Hollow Spherical Particles 4

The lignin or lignin-hemicellulose complex used in Reference Test Example 2 was used as a sample to prepare hollow spherical particles. Specifically, the preparation was performed as described below.

The lignin-saccharide complex or lignin powder was added to DMSO to a concentration of 10 w/v%, and homogeneously dissolved by ultrasonication. As a poor solvent, an ethanol/aqueous solution was added thereto in the direction of gradually decreasing the concentration of the organic solvent, followed by stirring, and the final organic solvent concentration was 30%.

The particle size and zeta potential of the obtained hollow spherical particles were measured by the electrical resistance nanopulse method (qNANO). The size distribution, concentration, and zeta charge of the nanoparticles were measured according to the principle of TRPS using a nanoparticle multianalyzer (produced by Izon Science and Meiwafosis Co., Ltd.) based on the electrical resistance nanopulse method (qNANO). 35 µL of particle solution was analyzed using NP200 and NP400 nanopores at 46-47 mm stretch conditions according to the manufacturer's protocol.

The particle size of the lignin-hemicellulose complex hollow spherical particles was smaller than that of lignin hollow spherical particles obtained under the same conditions. Similar tests were performed using different tree species from which the samples were derived, and similar results were obtained. The results are shown in Table 10.

**Table 10**

| **Sample name** | **Average particle size (nm)** | **Mode particle size (nm)** |
|---|---|---|
| **Eucalyptus-derived lignin capsules** | **404** | **278** |
| **Eucalyptus-derived lignin-polysaccharide complex capsules** | **283** | **246** |
| *Cryptomeria japonica***-derived lignin capsules** | **362** | **301** |
| *Cryptomeria japonica****-*derived lignin-polysaccharide complex capsules** | **285** | **247** |
| *Pinus densiflora***-derived lignin capsules** | **380** | **297** |
| *Pinus densiflora-***derived lignin-polysaccharide complex capsules** | **286** | **263** |

Further, the zeta potential measurement results revealed that the lignin-hemicellulose complex hollow spherical particles were cationic nanoparticles with a high positive zeta potential of 40 to 60 mV.

The surface charge of lignin-saccharide complex capsules was measured using a nanoparticle multianalyzer (produced by Izon Science and Meiwafosis Co., Ltd.) based on the electrical resistance nanopulse method (qNANO).

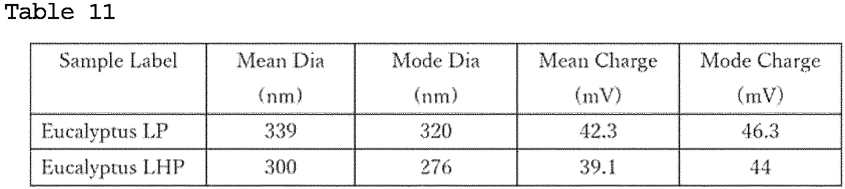

Table 11 shows the results of measuring the particle size and zeta potential of eucalyptus-derived lignin capsules (LP) and eucalyptus-derived lignin-saccharide complex capsules (LHP).

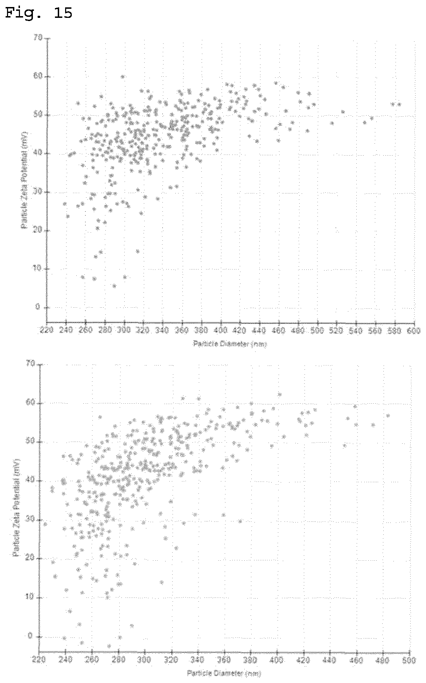

Fig. 15 shows the particle size and zeta potential distribution of eucalyptus-derived lignin capsules (LP, top) and eucalyptus-derived lignin-saccharide complex capsules (LHP, bottom). The particle size and zeta potential of each particle were obtained by the electrical resistance nanopulse method (qNANO) and plotted.

Scanning microscopy (SEM) images were obtained for the resulting hollow spherical particles. 0.2 mL of a solution containing the hollow spherical particles was pretreated with a nano-percolator (produced by JEOL Ltd.), and the hollow spherical particles remaining on the membrane were treated with a conductive coating using an osmium coater (produced by Meiwafosis Co., Ltd.) and observed with a field emission scanning electron microscope (S-4800, produced by Hitachi High-Tech Corporation).

Fig. 11-2 shows the scanning microscopy (SEM) images. Lignin capsules with a particle size of 100 nm to 600 nm and lignin-saccharide complex capsules with a particle size of 20 nm to 200 nm were identified. The SEM photographs show that the hollow spherical particles do not break and can maintain their shape even in a vacuum, indicating the stability of the particle structure. Since vesicle structures derived from natural molecules, such as liposomes and exosomes, are difficult to maintain their shape during normal SEM observation, which involves a vacuum-drying step, the structural stability is particularly noteworthy.

The lignin-saccharide complex capsules can have the ability to encapsulate highly hydrophilic drugs due to their properties as amphiphilic polymers. In fact, by the same test as in Reference Test Example 6, nucleic acid staining fluorescent dye DAPI [4,6-Diamidino-2-phenylindole, dihydrochloride] (produced by Dojindo Laboratories, CAS RN: 28718-90-3), chlorophyll (produced by Tokyo Chemical Industry Co., Ltd., CAS RN: 1406-65-1) as a dye, and iron porphyrin complex hemin (produced by Tokyo Chemical Industry Co., Ltd., CAS RN: 16009-13-5) as a metal complex were encapsulated. The prepared capsules were separated from low-molecular-weight components by dialysis, the dye absorption and absorption in the Soret-band and Q-band regions in the UV-vis spectrum were observed, and drug encapsulation was confirmed by microscopic observation (bright field, fluorescence). Violamo DNA Ladder Marker (VIO-1kb, produced by AS ONE Corporation) as nucleic acid DNA and Precision Plus Protein Marker (#1610377, produced by Bio-Rad) as protein were encapsulated. DNA was used after pre-conjugation with SYBR Green fluorescent dye. Drug encapsulation was confirmed by UV-vis spectroscopy and fluorescence microscopy in a similar manner.

Fig. 12-2 shows fluorescent micrographs (Ex 360 nm, Em 460 nm), bright-field micrographs, and electron micrographs of lignin-saccharide complex capsules encapsulating DAPI.

The fluorescence and bright-field micrographs were taken using an all-in-one fluorescence microscope (BZ-X810, produced by Keyence Corporation). 0.2 mL of a solution containing the DAPI-encapsulating lignin-saccharide complex was pretreated with a nano-percolator (produced by JEOL Ltd.), and the hollow spherical particles remaining on the membrane were treated with a conductive coating using an osmium coater (produced by Meiwafosis Co., Ltd.) and observed with a scanning electron microscope SEM (VE-9800, produced by Keyence Corporation). The accelerating voltage was 8 kV.

The lignin-saccharide complex capsules can exhibit long-wavelength fluorescence. Fig. 16 shows representative examples of 3D fluorescence spectra obtained in the same manner as in Reference Test Example 3.

### Example 2. Production of Hollow Spherical Particles 5

The lignin or lignin-hemicellulose complex used in Reference Test Example 2 was used as a sample to prepare hollow spherical particles in the same manner as in Example 1.

The average particle size of the obtained hollow spherical particles was measured in the same manner as in Example 1.

The stability of lignin hollow spherical particles obtained under the same conditions as those for the lignin-hemicellulose complex hollow spherical particles was compared and evaluated at a solute concentration of 0.5 w/v% using 30 v/v% ethanol/water as a solvent at room temperature.

In addition, the fine particle solution was heated and stirred, and the average particle size was measured after 0.5 hours.

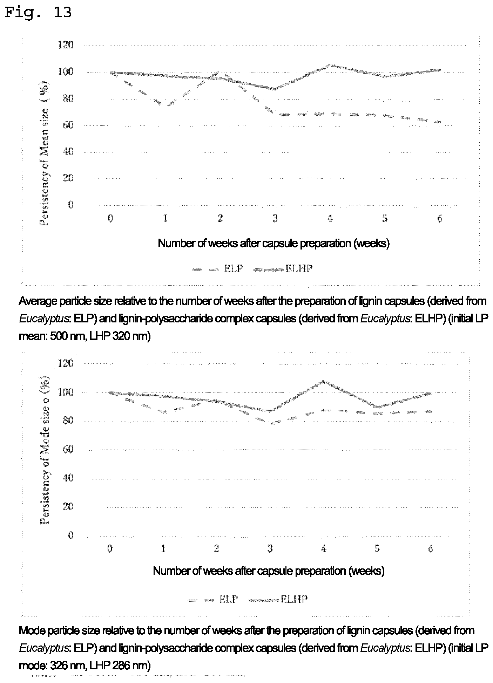

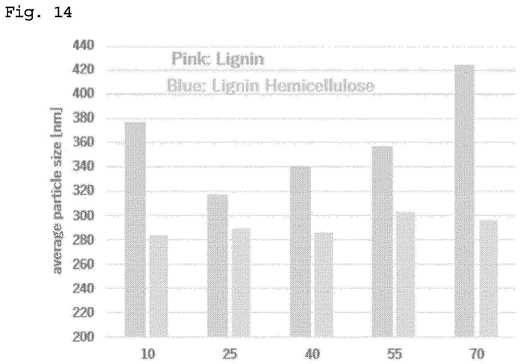

The particle size stability and temperature stability are shown in Figs. 13 and 14. The lignin-hemicellulose complex particles showed excellent properties in terms of dispersion retention ability over time, structural stability, and temperature stability. Specifically, as the dispersion retention ability and structural stability, the rate of change in number average particle size and mode average particle size over 6 weeks was within ±13%, and as the temperature stability, the rate of change in average particle size in the temperature range of 10°C to 70°C was within ±5%.

### Example 3. Experiment to Introduce Protein into Eucalyptus-Derived Lignin-Saccharide Complex Capsules

Lignin-saccharide complex capsules can be prepared under mild conditions and thus can have the ability to encapsulate biopolymers, such as proteins and nucleic acids. Polysaccharides are highly biocompatible and are thus promising candidates for DDS.

In general, liposomes and PEG-modified nanocarriers have low cellular uptake efficiency due to their negative surface charge. The lignin-saccharide complex capsules showed a high positive surface charge in water (weakly acidic) (zeta potential measurement results, mode average: +44 mV, particle size: 276 nm). On the other hand, under basic conditions (50 mM PBS, pH 11), they showed a negative surface charge (zeta potential measurement results, mode average: -21 mV, particle size: 302 nm). These characteristics have an additional function as a cationic drug carrier (DDS) with high cellular uptake efficiency. Furthermore, they are advantageous for drug introduction and nanocarrier retention in tumor tissue, which is in an acidic environment. In addition, they function as pH-responsive nanocarriers that can switch from a positive charge to a negative charge with almost no change in particle size.

### Example 4. Sustained Release Properties of Eucalyptus-Derived Lignin-Saccharide Complex Capsules by Glycolytic Enzyme

Bovine serum albumin (produced by Novagen) was used as protein. 100 µg/ml albumin was fluorescently labeled. Fluorescent labeling was performed using WSE-7010 EzLabel FluoroNeo (Atto Corporation). 10 µL of albumin solution (50 ug/ml, 0.5 µg) was added to 200 µL of lignin-saccharide complex (0.5 w/v, 35% v/v ethanol solution), and the resulting mixture was encapsulated into lignin-saccharide capsules, followed by filtration and observation using Keyence All-in-One Fluorescence Microscope BZ-X810. The encapsulation of fluorescently labeled protein was observed under a fluorescent microscope (equipped with a fluorescent filter for GFP).

Next, the polysaccharide-degrading enzyme hemicellulase (Hemicellulase Amano 90, produced by Amano Enzyme Inc.) was added to a final concentration of 1 mg/mL, followed by incubation at 50°C for 90 minutes. It was observed that fluorescence was no longer noticeable due to the enzyme treatment, and that the labeled protein leaked from the lignin-saccharide capsules and was released slowly.

### Example 5. Analysis of Hollow Spherical Particles

### 5-1. Method

### 5-1-1. Production of Hollow Spherical Particles

Eucalyptus lignin (PA1%AA, MW50, ACTN) and eucalyptus LCC lignin (PA1%AA, MW50→140, ACTN) were used. First, 10 w/v% lignin stock solutions dissolved in DMSO were diluted with DMSO to prepare lignin stock solutions of different concentrations. Five concentrations were prepared: 1.0 w/v%, 2.5 w/v%, 5.0 w/v%, 7.5 w/v%, and 10 w/v%. 10 µL of each concentration lignin solution was prepared in a 1.5 mL tube. The particle size was evaluated by the electrical resistance nanopulse method (qNANO) (NP400). Measurements were taken under conditions of Pressure 5 and Tension 47.01 cm. The samples were diluted with PBS buffer containing surfactant. The dilution ratios are shown in the table. 35 µL of sample dilution solution was applied at the time of measurement.

### 5-1-2. Examination of Temperature Conditions during Assembling

When fine particles were assembled by stirring and rotating reaction with an organic solvent, the effect of temperature conditions on the particle size and fine-particle concentration was examined. The samples used were eucalyptus lignin (PA1%AA, MW50, ACTN) and eucalyptus LCC lignin (PA1%AA, MW50-140, ACTN). 10 µL of 10 w/v% lignin stock solution dissolved in DMOS was dispensed into 1.5 mL tubes. 50 µL of EtOH at each concentration was used to mix with the lignin/DMSO solution; however, in order to perform the rotating reaction under high-temperature conditions, 60 µL of EtOH was used at each concentration, in consideration of solvent volatilization. The reaction was performed under five different temperature conditions: 10°C, 25°C, 40°C, 55°C, and 70°C. Before the rotating reaction, the temperature of the lignin stock solution and each EtOH solution was adjusted with an Eppendorf ThermoMixer so as to be similar to the temperature during the rotating reaction. The prepared fine particles were evaluated by the electrical resistance nanopulse method (qNANO) to compare the particle size and concentration.

### 5-1-3. Effect of Stirring Rotation Speed on Particle Size When Adding Organic Solvent

The effect of the solution stirring rotation speed conditions during the preparation of fine particles on the particle size of the fine particles was examined. The sample used was eucalyptus lignin (PA1%AA, MW50, ACTN). At that time, the rotation speed was set to five conditions: 300 rpm, 600 rpm, 900 rpm, 1200 rpm, and 1500 rpm. The particle size of lignin fine particles was measured by the electrical resistance nanopulse method (qNANO).

In addition, fine particles prepared under rotating reaction conditions of 300 rpm were reacted at 1500 rpm to examine the change in particle size. 180 µL of a sample prepared by mixing at 300 rpm was mixed at 25°C and 1500 rpm for 5 minutes (300 to 1500 rpm sample).

Next, the effect of the rotation speed on the particle size of fine particles was evaluated using eucalyptus LCC lignin (PA1%AA, MW50, ACTN). The rotation speed was set to four conditions: 300 rpm, 500 rpm, 700 rpm, and 900 rpm. The particle size of lignin fine particles was measured by the electrical resistance nanopulse method (qNANO).

### 5-1-4. Comparison of Effect of Centrifugal Direction on Particle Size of Fine Particles

The equipment and rotation direction when mixing a lignin solution and an organic solvent were examined. The effects of vertical rotation, horizontal rotation, and the difference in equipment used on the particle size of lignin fine particles were examined. A 10 w/v% lignin/DMSO solution was prepared as in Experimental Method 1-1, and 80 v/v%, 40 v/v%, and 20 v/v% aqueous ethanol solutions were prepared. When mixing the lignin solution and each aqueous ethanol solution, 50 µL of the aqueous ethanol solution was added stepwise, starting with the more concentrated solution, and centrifugation was repeated. Centrifugation was performed by using 1000 rpm of centrifugation in the X-axis direction using an Eppendorf ThermoMixer, and 1000 rpm of stirring in the X-axis and Y-axis directions using Lab Serb. The particle size of the resulting lignin fine particles was measured by the electrical resistance nanopulse method (qNANO) using NP400.

5-1-5. Evaluation of Fine Particle Size under Different pH Buffer

### Conditions

When preparing lignin fine particles, whether there would be differences in the particle size of the fine particles and the ease of producing the fine particles depending on the type of buffer added was examined. The samples used were eucalyptus lignin (PA1%AA, MW50, ACTN) and eucalyptus LCC lignin (PA1%AA, MW50→140, ACTN). Three types of aqueous ethanol solutions were prepared: 80 v/v%, 40 v/v, and 20 v/v%, with 50 µL for each sample. In this case, ethanol was diluted with each buffer. The buffers were prepared by diluting 1 M MOPS/NaOH buffer (pH 6.4), 1 M PIPES/NaOH buffer (pH 6.2), and 1 M HEPES/NaOH (pH 8.2) to a final concentration of 50 mM. The particle size of the lignin fine particles was evaluated by the electrical resistance nanopulse method (qNANO) using NP400. Further, the particle size of the LCC lignin fine particles was evaluated by the electrical resistance nanopulse method (qNANO) using NP200.

### 5-1-6. Examination of Organic Solvent to Be Added

The effects of organic solvents used in the assembling of lignin on the particle size of fine particles and the ease with which lignin was assembled were compared. The samples used were eucalyptus lignin (PA1%AA, MW50, ACTN) and eucalyptus LCC lignin (PA1%AA, MW50→140, ACTN). Lignin was assembled under the same conditions as in Experimental Method 1-1. The organic solvents added were methanol, ethanol, 1-propanol, and 1-buthanol, each diluted with pure water to concentrations of 80 v/v%, 40 v/v, and 20 v/v%. Using the NP400 nanopore, particle size evaluation and concentration difference measurement were performed by the electrical resistance nanopulse method (qNANO).

### 5-1-7. Assembling of Lignin Using Different Resins

Whether lignin fine particles could be produced not only from hardwood such as eucalyptus, but also from softwood such as *Cryptomeria japonica* and *Pinus densiflora,* was examined. Seven types of resins were used: eucalyptus lignin (PA1%AA, MW50, ACTN), eucalyptus LCC lignin (PA1%AA, MW50→140, ACTN), *Cryptomeria japonica* lignin (H₂O₂ 3.2%+AA, MW50, 80DOX), *Cryptomeria japonica* LCC lignin (H₂O₂ 3.2%+AA, MW50→140, 80DOX), *Pinus densiflora* (H₂O₂ 3.2%+AA, MW50, ACTN), *Pinus densiflora* LCC lignin (H₂O₂ 3.2%+AA, MW50→140, ACTN), and bamboo lignin (MS130-5). Fine particles were prepared, and the particle size was measured by the electrical resistance nanopulse method (qNANO) using NP400.

**Table 12**

| **Dilution ratio during measurement of lignin fine particles by electrical resistance nanopulse method (qNANO)** | |
|---|---|
| **Tree species** | **Dilution ratio (times)** |
| **Eucalyptus lignin (PA1AA, MW50, ACTN)** | **20** |
| **Eucalyptus LCC lignin (PA1AA, MW50→140, ACTN)** | **10** |
| *Cryptomeria japonica* **lignin (H₂O₂ 3.2%+AA, MW50, 80DOX)** | **1.1** |
| *Cryptomeria japonica* **LCC lignin (H₂O₂ 3.2%+AA, MW50→140, 80DOX)** | **5.0** |
| *Pinus densiflora* **(H₂O₂ 3.2%+AA, MW50, ACTN)** | **1.2** |
| *Pinus densiflora* **LCC lignin (H₂O₂ 3.2%+AA, MW50→140, ACTN)** | **5.0** |
| **Bamboo lignin (MS130-5)** | **10** |

### 5-1-8. Evaluation of Fine Particles by Electrical Resistance Nanopulse Method (qNANO)

For both NP200 and NP400, the nanopore stretch condition was adjusted to 47 mm. The voltage was adjusted so that the current value upon addition of buffer was approximately 120 nA. First, after calibration, sample measurements were performed. The calibration sample used was as follows: Mean Dia: 350 nm, Mode Dia: 330 nm, and concentration: 8.4E+11 particle/mL. The sample was diluted 500 times with PBS buffer containing surfactant, and 35 µL was applied for measurement. Subsequently, sample measurements were performed. The sample was diluted with PBS buffer, and 35 µL was applied for measurement. The measurement time was 10 minutes or less. When the current value was unstable, a click was performed.

### 5-1-9. Particle Size Evaluation by Dynamic Light Scattering Method

The particle size was evaluated using SZ-100 Nanoparticle Size Analyzer (HORIBA). Measurements were performed using a four-sided transmission quartz cell. For sample preparation, a lignin fine particle sample was diluted 100 times with pure water to prepare 2 mL. The measurement time was set to auto mode, and the peak tendency was measured as the polydispersion and standard peak. Measurements were performed three or more times, and the particle size was evaluated by calculating the average value.

### 5-1-10. Particle Size Evaluation Using Interference Microscope

The particle size of fine particles was evaluated using a nanoparticle imaging analyzer (VIDEO DROP, Meiwafosis Co., Ltd.). The prepared lignin fine particles were diluted 100 times with pure water and measured. During the measurement, the presence or absence of aggregates was confirmed on the monitor. At the time of measurement, 8 µL of the diluted solution was applied, and the count of samples was measured until 300 or more.

### 5-2. Results

### 5-2-1. Particle Size Distribution Analysis

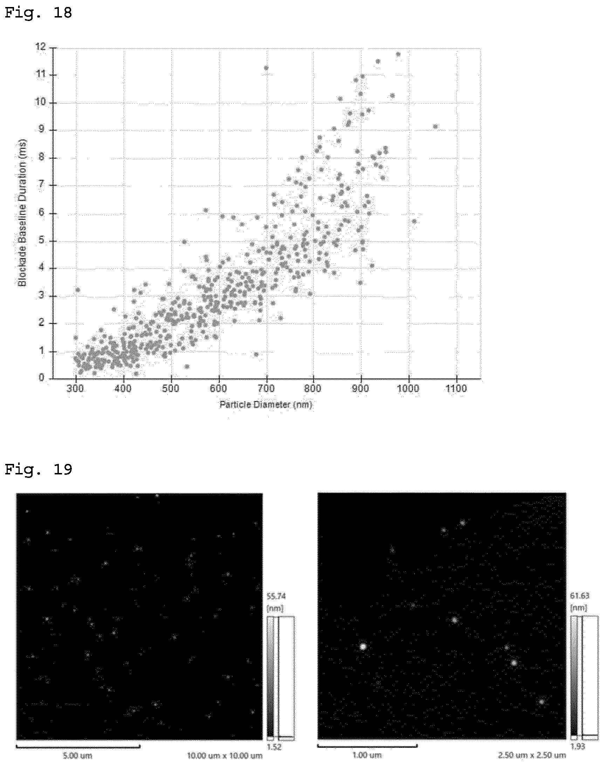

Fig. 18 shows the analysis results of particle size and pore passing time for each fine particle. The plot distribution on the diagonal indicates that the fine particles have high sphericity (symmetry).

### 5-2-2. Observation of Nanoparticles Using Microscope

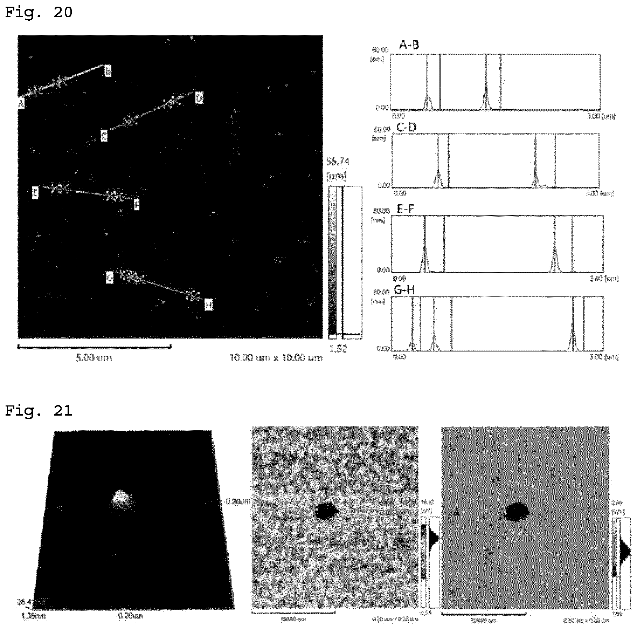

An ethanol/aqueous solution was gradually added to eucalyptus lignin (PA1%AA, MW50, ACTN) to create poor solvent conditions as a whole, and lignin fine particles were prepared. The shape of the nanoparticles was observed using a scanning probe microscope SPM-Nanoa (Shimadzu Corporation) and their physical properties were evaluated. The results of SPM confirmed that fine particles of 100 nm or less were produced (Table 13). It was also confirmed that the nanoscale fine particles were spherical in shape (Fig. 19). The results of the physical property analysis of the nanoparticles confirmed that the lignin fine particles had a lower affinity for the probe than mica, and were relatively soft (Fig. 21).

**Table 13**

| **SPM nanoparticle 3D height analysis results** | | | |
|---|---|---|---|
| **Straight line** | **Height evaluation (red mark) (nm)** | **Height evaluation (green mark) (nm)** | **Height evaluation (blue mark) (nm)** |
| A-B | 23.03 | 35.21 | NA |
| C-D | 30.72 | 22.92 | NA |
| E-F | 34.24 | 38.42 | NA |
| G-H | 15.40 | 25.69 | 41.52 |

**Table 14**

| **SPM nanoparticle 3D slope analysis results** | | | |
|---|---|---|---|
| **Straight line** | **Height evaluation (red mark) (°)** | **Height evaluation (green mark) (°)** | **Height evaluation (blue mark) (°)** |
| A-B | 6.07 | 9.07 | NA |
| C-D | 10.84 | 4.46 | NA |
| E-F | 6.96 | 8.60 | NA |
| G-H | 7.30 | 5.63 | 14.52 |

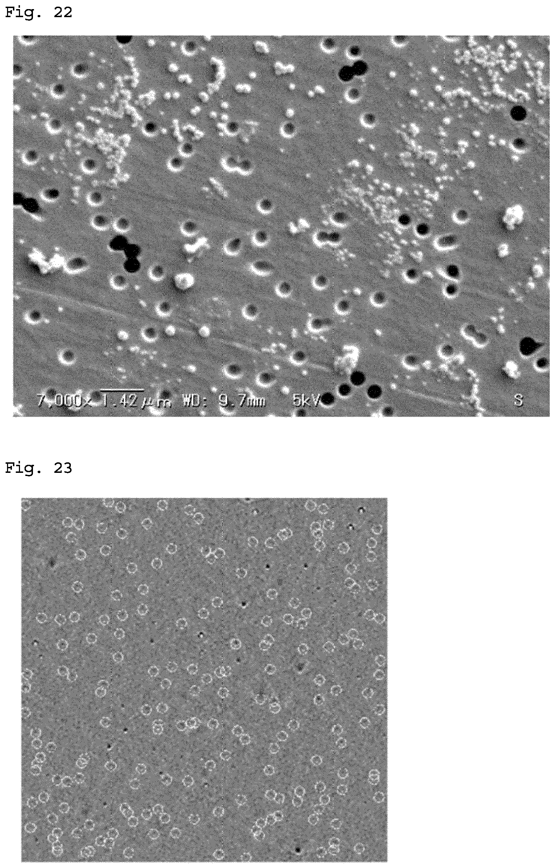

Subsequently, LCC lignin nanoparticles prepared from eucalyptus LCC lignin (PA1%AA, MW50→140, ACTN) were observed using a 3D real surface view microscope. The prepared fine particles were dried using a nano-percolator, then coated with osmium, and observed using a 3D real surface view microscope. The experimental results showed that spherical fine particles were observed (Fig. 22).

The particle size of nanoscale LCC lignin fine particles prepared from eucalyptus LCC lignin (PA1%AA, MW50→140, ACTN) was evaluated using an interference microscope and DLS. The experimental results showed that the median diameter was 98 nm and the mode diameter was 105 nm for the interference microscope, and 90.7 nm and 85.0 nm for DLS (Table 15). The results of the interference microscope experiment also confirmed that granular nanoparticles were generated (Fig. 23).

**Table 15**

| **Evaluation of particle size of LCC nanoparticles** | | |
|---|---|---|
| **Analysis method** | **Median diameter** (nm) | **Mode diameter** (nm) |
| **Interference microscope** | 98 | 105 |
| **Dynamic light scattering method** | 90.7 | 85.0 |

### 5-2-3. Evaluation of Dispersibility of Lignin Fine Particles

### 5-2-3-1. Zeta Potential Measurement by Electrical Resistance Nanopulse Method (qNANO)

Fine particles were prepared from eucalyptus lignin (PA1%AA, MW50, ACTN) and eucalyptus LCC lignin (PA1%AA, MW50→140, ACTN) using a phosphate buffer at a pH from 3 to 11 in increments of 2, and the zeta potential of the fine particles was measured by the electrical resistance nanopulse method (qNANO) using NP400. First, the calibration was measured. The measured concentrations for calibration were in the order of V₁P₂, V₁P₁, V₂P₁, and V₃P₁, confirming that the calibration was accurate. Furthermore, the calibration particles did not clog pores, and the fine particle count showed a good linear proportional relationship with the measurement time. First, the particle size of the calibration particles was measured. The measured median diameter was 330 nm, confirming that the measurement was accurately calibrated.

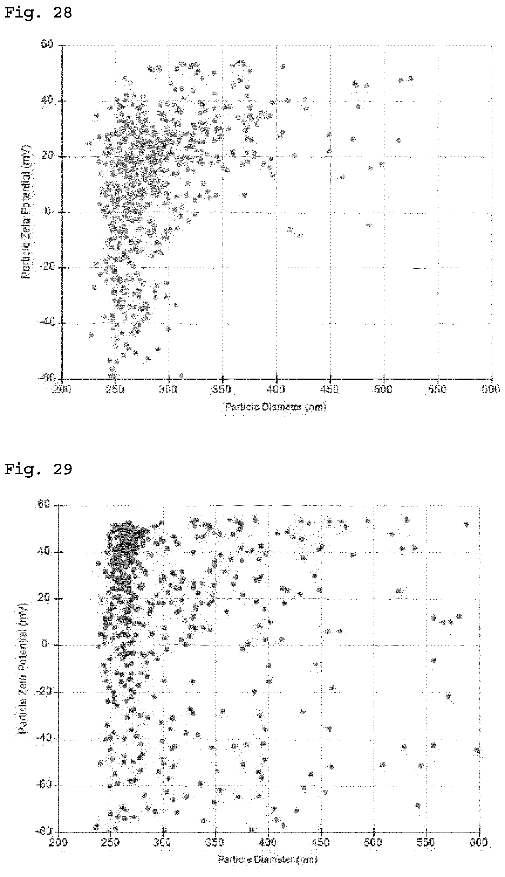

The results of measuring the zeta potential of lignin fine particles showed that the mean absolute value was positive at 30.5 mV under acidic conditions at a pH of 3 and negative at - 26.6 mV under alkaline conditions at a pH of 11 (Table 16). Under neutral conditions, it was confirmed that fine particles with a particle size of more than 250 nm showed a positive zeta potential, while fine particles with a particle size of 300 nm or less were both negatively and positively charged (Fig. 28). From this, it was considered that there may be a correlation between the zeta potential and the particle size.

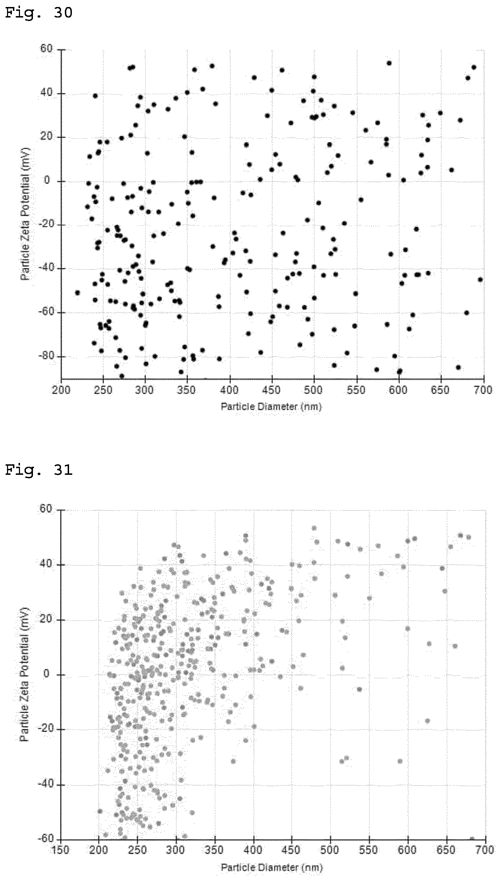

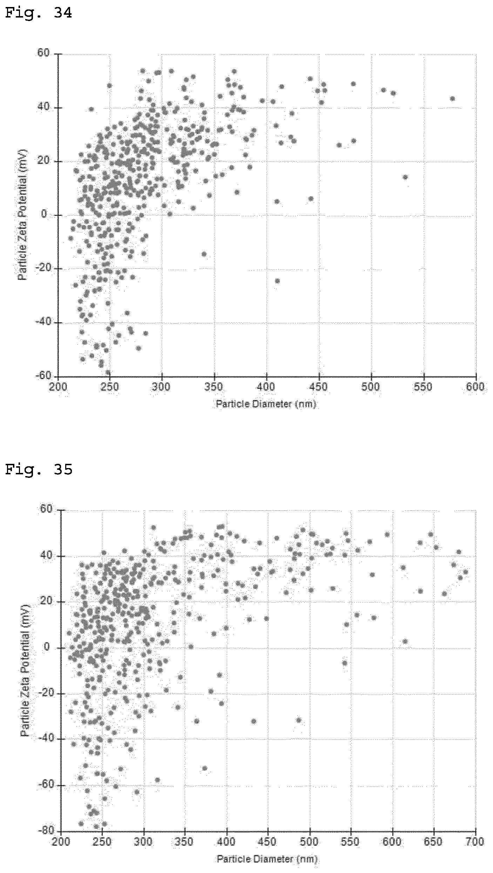

In the LCC lignin fine particles, many fine particles were positively charged at all pH conditions (Table 17). At a pH condition of 7, fine particles with a particle size of 300 nm or less that were both positively and negatively charged were confirmed, similar to polysaccharide-free lignin fine particles (Fig. 30). As the pH became more alkaline from here, the plots tended to expand vertically (Figs. 34 and 35).

**Table 16**

| **Measurement results of particle size and zeta potential of lignin fine particles for each pH** | | | | |
|---|---|---|---|---|
| **Sample pH conditions** | **Average particle size (nm)** | **Mode particle size (nm)** | Mean Charge (mV) | Mode Charge (mV) |
| pH3 | 448 | 283 | 30.5 | 32.8 |
| pH5 | 302 | 248 | 6.55 | 28.1 |
| pH7 | 293 | 254 | 7.97 | 22.6 |
| pH9 | 321 | 270 | 6.68 | 47.8 |
| pH11 | 426 | 259 | -26.6 | -31.2 |

**Table 17**

| **Measurement results of particle size and zeta potential of LCC lignin fine particles for each pH** | | | | |
|---|---|---|---|---|
| **Sample pH conditions** | **Average particle size (nm)** | **Mode particle size (nm)** | Mean Charge (mV) | Mode Charge (mV) |
| pH3 | 308 | 250 | -4.01 | 18.8 |
| pH5 | 262 | 228 | -1.63 | 18.9 |
| pH7 | 288 | 239 | 22.4 | 31.5 |
| pH9 | 287 | 240 | 11.8 | 24.7 |
| pH11 | 325 | 259 | 10.2 | 32.3 |

### 5-2-3-2. Zeta Potential Measurement Using DLS

Fine particles were prepared from eucalyptus lignin (PA1%AA, MW50, ACTN) and eucalyptus LCC lignin (PA1%AA, MW50-140, ACTN) using a phosphate buffer at a pH from 1 to 13 in increments of 2, and the zeta potential of the fine particles was measured using DLS.

The experimental results showed that the average zeta potential of the lignin fine particles became more negative as the pH increased from acidic to alkaline (Table 18). At pH conditions of 1 and 3, the most frequent zeta potentials were positive at 1.67 mV and 1.48 mV, respectively. From the neutral conditions, multiple zeta potential peaks were observed instead of a single peak. On the other hand, the average zeta potential of the LCC lignin fine particles was positive at 2.81 mV under acidic conditions at a pH of 1, but was negative at a pH of 3 to alkaline conditions. In particular, at a pH of 9, the absolute value of average zeta potential was -75.6 mV, which was larger than that of polysaccharide-free lignin fine particles (Table 19).

**Table 18**

| **Measurement results of zeta potential of lignin fine particles using DLS** | | | | | | | |
|---|---|---|---|---|---|---|---|
| **Sample pH** | pH1 | pH3 | pH5 | pH7 | pH9 | pH11 | pH13 |
| **Average zeta potential** (mV) | 0.563 | -1.1 | -9.51 | -15.0 | -26.4 | -48.7 | -40.5 |
| **Maximum zeta potential** (mV) | 1.76 | 1.48 | -9.84 | -10.0 | -40.1 | -55.7 | -49.2 |
| | | | | 12.2 | -19.1 | -24.0 | -27.9 |
| | | | | | | 0.034 | |

**Table 19**

| **Measurement results of zeta potential of LCC lignin fine particles using DLS** | | | | | | | |
|---|---|---|---|---|---|---|---|
| **Sample pH** | pH1 | pH3 | pH5 | pH7 | pH9 | pH11 | pH13 |
| **Average zeta potential** (mV) | 2.81 | -32.1 | -54.2 | -71.1 | -75.6 | -54.2 | -73.7 |
| **Maximum zeta potential** (mV) | -0.606 | -34.7 | -55.2 | -44.0 | -29.2 | -36.7 | -46.0 |
| | | | | -65.7 | -59.1 | -81.5 | -68.4 |
| | | | | -78.4 | -81.5 | | -83.4 |

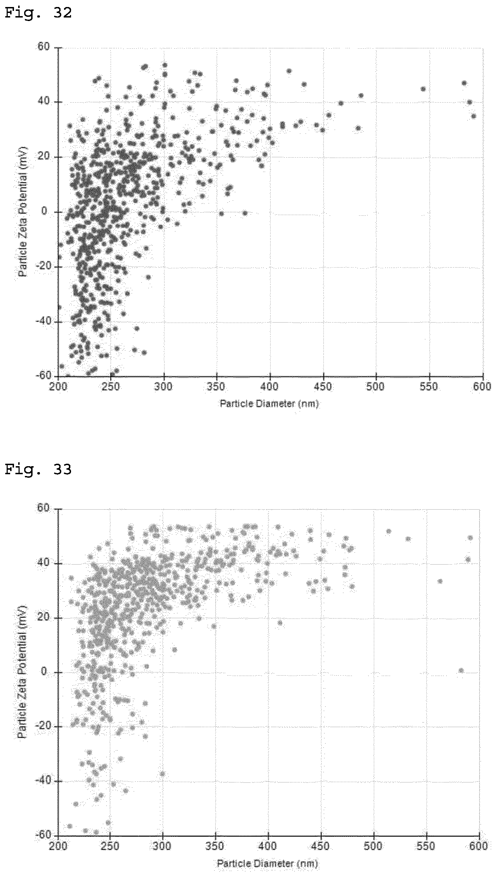

From the results of zeta potential measurements by the electrical resistance nanopulse method (qNANO), it was considered that fine particles with a smaller particle size are more likely to become negatively charged, and that there is a correlation between the particle size and the zeta potential (Fig. 33). Since the analytical range of particle size is different between the electrical resistance nanopulse method (qNANO) and the zeta potential, assuming that the difference was due to the zeta potential, the particle size of LCC lignin fine particles was measured at a pH of 7 by DLS. As a result, the median diameter was 113 nm, the mode particle size was 78.7 nm, and the observed fine particles were more nanoscale than the mode particle size of 239 nm measured by the electrical resistance nanopulse method (qNANO) (Fig. 33).

### 5-2-4. Evaluation of Particle Size Persistence of Lignin Fine Particles

The degree of change in the particle size of lignin fine particles with respect to the elapsed time was measured. Lignin fine particles were prepared using eucalyptus lignin (PA1%AA, MW50, ACTN) and eucalyptus LCC lignin (PA1%AA, MW50→140, ACTN) tree species, and the degree of change in average particle size and mode particle size over 6 weeks was measured. The particle size of the lignin fine particles at week 0 was regarded as 100%, and the experimental results showed how long the particle size was maintained after the following week. For the LCC lignin fine particles, the change in average particle size was 15% or less even after 6 weeks (Table 20). The persistence of mode particle size after 6 weeks was almost 100% for both the lignin fine particles and the LCC lignin fine particles (Table 21).

**Table 20**

| **Shape retention of average particle size over time** | | | | | | |
|---|---|---|---|---|---|---|
| **Tree species/time elapsed (weeks)** | **1** | **2** | **3** | **4** | **5** | **6** |
| **Lignin fine particles (%)** | **74** | **101** | **68.2** | **69.2** | **67.6** | **62.8** |
| **LCC lignin fine particles (%)** | **86.5** | **95.1** | **77.9** | **88** | **85.3** | **86.8** |

**Table 21**

| **Continuity of mode particle size over time** | | | | | | |
|---|---|---|---|---|---|---|
| **Tree species/time elapsed (weeks)** | **1** | **2** | **3** | **4** | **5** | **6** |
| **Lignin fine particles (%)** | **97.5** | **95.3** | **87.5** | **106** | **96.9** | **102** |
| **LCC lignin fine particles (%)** | **97.6** | **94.1** | **87.1** | **108** | **89.9** | **99.7** |

**Table 22**

| **Measurement results of average particle size over time** | | | | | | | |
|---|---|---|---|---|---|---|---|
| **Tree species/time elapsed (weeks)** | **0** | **1** | **2** | **3** | **4** | **5** | **6** |
| **Lignin fine particles (nm)** | **500** | **370** | **507** | **341** | **346** | **338** | **314** |
| **LCC lignin fine particles (nm)** | **320** | **312** | **305** | **280** | **338** | **310** | **326** |

**Table 23**

| **Measurement results of mode particle size over time** | | | | | | | |
|---|---|---|---|---|---|---|---|
| **Tree species/time elapsed** (weeks) | 0 | 1 | 2 | 3 | 4 | 5 | 6 |
| **Lignin fine particles** (nm) | 326 | 282 | 310 | 254 | 287 | 278 | 285 |
| **LCC lignin fine particles** (nm) | 286 | 279 | 269 | 249 | 309 | 257 | 285 |

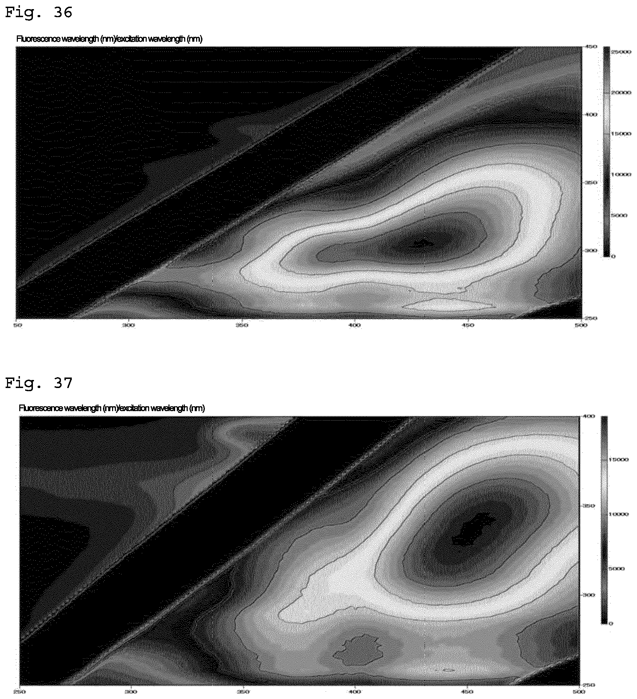

Using eucalyptus LCC lignin (PA1%AA, MW50→140, ACTN), fine particles were prepared by combining eosin molecules as a drug model compound for the assembling of lignin. When the fluorescence excitation wavelengths of the prepared eosin complex fine particles and eosin-free fine particles were measured, a shift in the peak wavelength was confirmed (Figs. 36 and 37). The eosin-free fine particles had a peak fluorescence wavelength of 428 nm and an excitation wavelength of 310 nm, while the eosin complex fine particles had a peak fluorescence wavelength of 456 nm and an excitation wavelength of 346 nm.

### 5-2-5. Combined Testing for Pharmaceutical Applications

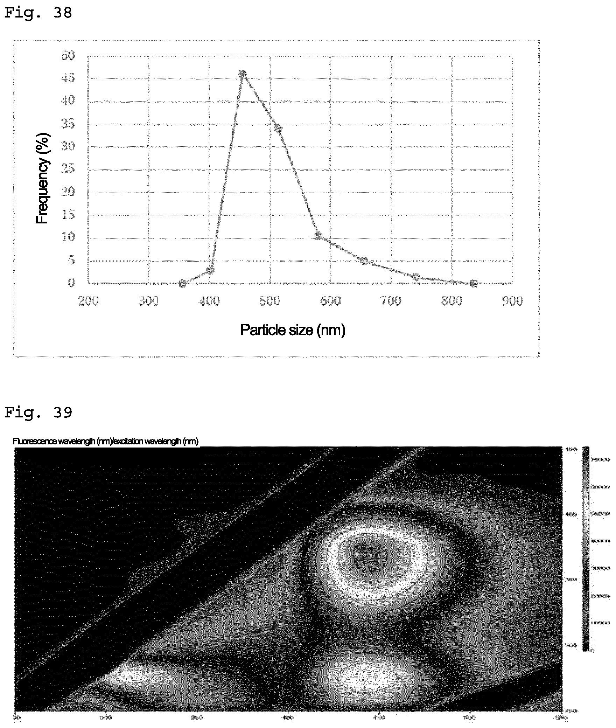

Fine particles were prepared by combining eucalyptus LCC lignin (PA1%AA, MW50→140, ACTN) with diethyl sebacate, which is used in medicines and cosmetics. Diethyl sebacate is generally used to promote the penetration of fat-soluble substances into the stratum corneum and hair, and used as an emulsifier. The particle size of the fine particles prepared was measured by the electrical resistance nanopulse method (qNANO) using NP400 and by DLS. Each measurement was performed twice, and the average values are shown in Table 24. The results confirmed that a large number of fine particles of approximately 300 nm to 400 nm were produced. Further, the fluorescence excitation spectrum was measured. The experimental results showed that compared with the results of the fluorescence excitation spectrum of fine particles prepared from an ethanol aqueous solution (Fig. 36), both the fluorescence and excitation wavelengths showed peaks on the longer wavelength side. In addition, two peaks were confirmed at a fluorescence wavelength of 450 nm and excitation wavelengths of 279 nm and 370 nm (Fig. 39).

**Table 24**

| **Evaluation of particle size of diethyl sebacate complex fine particles** | | |
|---|---|---|
| **Measurement method** | **Median diameter** (nm) | **Mode diameter** (nm) |
| qNano | 373 | 304 |
| DLS | 462 | 459 |

### 5-2-6. Evaluation of Effect of Temperature Change on Fine Particles

### 5-2-6-1. Particle Size Evaluation Due to Temperature Change

The degree of change in the particle size of lignin fine particles due to continuous temperature changes was measured. Lignin fine particles were prepared using eucalyptus lignin (PA1%AA, MW50, ACTN) and eucalyptus LCC lignin (PA1%AA, MW50→140, ACTN) tree species, and the degree of change in particle size was measured by DLS while increasing the temperature stepwise by 10°C. The experimental results showed that there was no significant change in the particle size of the lignin fine particles even when the temperature was increased after the fine particles were prepared (Table 25). On the other hand, the average particle size of the LCC lignin fine particles became smaller by about 10 nm when the temperature was increased from 20°C to 60°C (Table 26).

**Table 25**

| **Evaluation of particle size of lignin fine particles due to temperature change** | | | | | | |
|---|---|---|---|---|---|---|
| **Temperature (°C)** | 10 | 20 | 30 | 40 | 50 | 60 |
| **Median diameter (nm)** | 145 | 145 | 152 | 152 | 148 | 135 |
| **Mode diameter (nm)** | 134 | 128 | 150 | 150 | 136 | 126 |

**Table 26**

| **Evaluation of particle size of LCC lignin fine particles due to temperature change** | | | | | | |
|---|---|---|---|---|---|---|
| **Temperature (°C)** | 10 | 20 | 30 | 40 | 50 | 60 |
| **Median diameter (nm)** | 70.2 | 67.8 | 63.3 | 63.7 | 56.3 | 57.3 |
| **Mode diameter (nm)** | 65.1 | 64.6 | 62.3 | 61.8 | 56.3 | 57.2 |

### 5-2-6-2. Zeta Potential Evaluation Due to Change

The degree of change in the zeta potential of lignin fine particles due to continuous temperature changes was measured. Lignin fine particles were prepared using eucalyptus lignin (PA1%AA, MW50, ACTN), and the zeta potential was continuously measured while increasing the temperature stepwise by 10°C. The experimental results showed that the zeta potential value increased as the temperature approached room temperature compared to low-temperature conditions (Table 27).

**Table 27**

| **Evaluation of zeta potential of LCC nanoparticles due to temperature change** | | | | | | |
|---|---|---|---|---|---|---|
| **Temperature (°C)** | 10 | 20 | 30 | 40 | 50 | 60 |
| **Zeta potential (mV)** | -7.20 | -12.4 | -15.1 | -10.4 | -15.6 | -11.9 |

### Test Example X. Preparation and Analysis of Water-in-Oil (W/O) and Oil-in-Water (O/W) Self-Assembled Bodies

Preparation and analysis of water-in-oil (W/O) and oil-in-water (O/W) self-assembled bodies in lignin-saccharide complexes

A lignin-saccharide complex isolated by the A-APA method (microwave treatment at 140°C for 10 minutes, acetic acid-peracetic acid 1% or acetic acid-0.2M hydrogen peroxide conditions) was further dispersed in an aqueous solution, and insoluble and soluble parts were separated. The insoluble part was used as the raw material for O/W type, and the soluble part was used as the raw material for W/O type. Table 28 shows the results of separation using an aqueous solution containing 10% acetic acid. The saccharide unit content (%) per 100 aromatic rings was calculated from the HSQC NMR spectrum as the percentage of saccharide units to aromatic ring units (calculated from the correlation signal integrated value of the anomeric carbon).

**Table 28**

| | **Raw material plant species** | **Saccharide unit content per 100 aromatic ring units (%)** | **Yield per lignin-saccharide complex (before fractionation) (wt.%)** | **Yield per raw material (wood flour etc.) (wt.%)** |
|---|---|---|---|---|
| **Aqueous solution (10% v/v acetic acid water) soluble part lignin-saccharide complex: water-in-oil (W/O) type** | ***Chamaecyparis obtusa*** | **169** | **45** | **6** |
| | ***Pinus densiflora*** | **309** | **54** | **9** |
| | ***Phyllostachys edulis*** | **207** | **47** | **12** |
| | ***Fagus crenata*** | **152** | **48** | **13** |
| | ***Eucalyptus*** | **188** | **43** | **12** |
| **Aqueous solution (10% v/v acetic acid water) insoluble part lignin-saccharide complex: oil-in-water (O/W) type** | ***Chamaecyparis obtusa*** | **4** | **50** | **6** |
| | ***Pinus densiflora*** | **3** | **49** | **8** |
| | **Sugarcane (bagasse)** | **31** | **33** | **4** |
| | ***Phyllostachys edulis*** | **11** | **51** | **13** |
| | ***Faqus crenata*** | **3** | **43** | **11** |
| | ***Eucalyptus*** | **6** | **50** | **14** |

A 10 wt% aqueous solution of the lignin-polysaccharide complex (W/O type) was prepared, and as a separating solvent, 200 µL of 1-butanol (FUJIFILM Wako Pure Chemical Corporation, special grade) was added to 10 µL of the aqueous solution. The addition was carried out in the cleaning tank of an ultrasonic cleaner while applying ultrasonic waves at a frequency of 28 kHz, and a self-assembled body was obtained.

The oil-in-water (O/W) type was prepared in the same manner as in other descriptions.

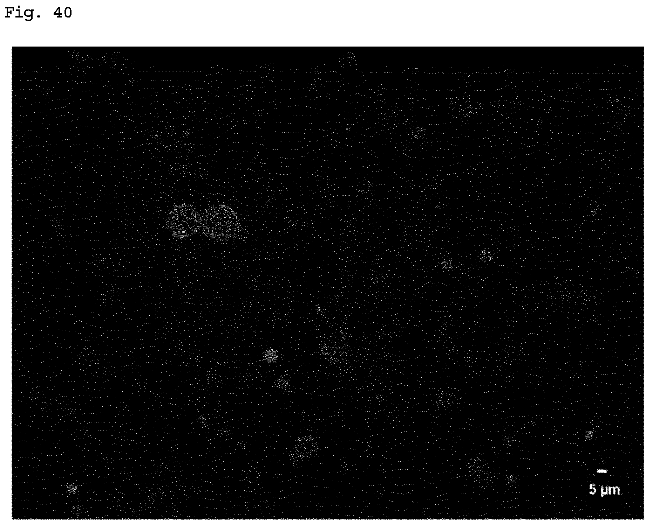

The structure of the self-assembled body was measured using a fluorescence microscope (All-in-One Fluorescence Microscope BZ-X810, produced by Keyence Corporation). Fig. 40 shows a photograph of water-in-oil (W/O) lignin-saccharide complex hollow spherical particles derived from *Pinus densiflora.*

## Claims

1. A hollow spherical particle that is a self-assembled body of a lignin-saccharide complex, the lignin-saccharide complex having at least one bond selected from the group consisting of an α-ether bond, an α-ester bond, and a γ-ester bond between lignin and polysaccharide.

2. The hollow spherical particle according to claim 1, wherein the constituent ratio of polysaccharide to lignin in the lignin-saccharide complex, which is a ratio obtained by dividing the number of monomer units of polysaccharide by the number of monomer units of lignin, is 0.1 to 10.

3. The hollow spherical particle according to claim 1, wherein the lignin-saccharide complex has a molecular weight (Mw) of 2,000 to 10,000 and a polydispersity (Mw/Mn) of 2.5 or less.

4. The hollow spherical particle according to claim 1, which is a population of hollow spherical particles composed of a lignin-saccharide complex and having an average particle size of 200 nm or more, and which has a positive average zeta potential at a pH of 7 and room temperature.

5. The hollow spherical particle according to claim 1, which is a population of hollow spherical particles composed of a lignin-saccharide complex and having an average particle size of less than 200 nm, and which has a negative average zeta potential at a pH of 7 and room temperature.

6. The hollow spherical particle according to claim 1, which has a number average particle size of 1 µm or less.

7. The hollow spherical particle according to claim 1, comprising an active ingredient.

8. The hollow spherical particle according to claim 7, wherein the active ingredient is at least one member selected from the group consisting of dyes, agricultural chemicals, fertilizers, pharmaceuticals, cosmetic ingredients, fragrances, fluorescent agents, paints, proteins, antibodies, nucleic acids, vaccines, lipids, hormones, sugars, glycosides, surfactants, adhesives, vitamins, coenzymes, minerals, cells, organelles, low-molecular-weight compounds, high-molecular-weight compounds, metal complexes, liposomes, and hydrogels.

9. The hollow spherical particle according to claim 1, which exhibits long-wavelength fluorescence at 450 nm or more.

10. A drug delivery or introducing agent comprising the hollow spherical particle according to any one of claims 1 to 9.

11. The drug delivery or introducing agent according to claim 10, wherein the drug is nucleic acid.

12. An ultraviolet absorber comprising the hollow spherical particle according to any one of claims 1 to 9.

13. A light resistance imparting agent comprising the hollow spherical particle according to any one of claims 1 to 9.

14. An anti-Stokes fluorescent agent comprising the hollow spherical particle according to any one of claims 1 to 9.
